# EUROPEAN PATENT SPECIFICATION

(11) **EP 1 144 605 B1**
(45) Date of publication and mention of the grant of the patent: **16.12.2009**
(21) Application number: 00901731.0
(22) Date of filing: 26.01.2000
(51) Int. Cl.: C12N 9/78, C12N 15/63, C07K 16/40, A01K 67/00, C12Q 1/34, A61P 3/00, A61P 13/00, A61P 9/00, A61P 25/18, A61P 21/00, A61P 19/00

(54) **DIMETHYLARGININE DIMETHYLAMINOHYDROLASES**
DIMETHYLARGININ-DIMETHYLAMINOHYDROLASEN
DIMETHYLARGININE DIMETHYLAMINOHYDROLASES

(30) Priority: 26.01.1999 GB 9901705; 04.06.1999 GB 9913066
(43) Date of publication of application: 17.10.2001
(73) Proprietor: UNIVERSITY COLLEGE LONDON, London WC1E 6BT (GB)
(72) Inventor: VALLANCE, Patrick John Thompson, St. Martins House, London W1P 9LN (GB); LEIPER, James Mitchell, St. Martins House, London W1P 9LN (GB); WHITLEY, Guy St. John, St. George's Hospital, Cranmer Terrace, London SW17 0RE (GB); CHARLES, Ian George, St. Martins House, London W1P 9LN (GB)
(74) Representative: Woods, Geoffrey Corlett
(86) International application number: PCT/GB2000/000226
(87) International publication number: WO 2000/044888

(56) References cited:
- WO-A-00/28045
- WO-A-98/07868
- WO-A-99/55858
- SPANJAARD R A ET AL: "Clone 10d/BM28 (CDCL1), an early S-phase protein, is an important growth regulator of melanoma." CANCER RESEARCH. 15 NOV 1997, vol. 57, no. 22, 15 November 1997 (1997-11-15), pages 5122-5128, XP001194462 ISSN: 0008-5472
- DATABASE EMBL 18 April 1997 (1997-04-18), ADAMS M.D. ET AL.: "EST182406 Jurkat T-cells VI Homo sapiens cDNA 5' end" XP002287346 Database accession no. AA311681
- LEIPER J M ET AL: "Identification of two human dimethylarginine dimethylaminohydrolases with distinct distribution and homology with microbial arginine deiminases" BIOCHEMICAL JOURNAL, PORTLAND PRESS, LONDON, GB, vol. 343, 1 October 1999 (1999-10-01), pages 209-214, XP002140903 ISSN: 0264-6021
- DATABASE EMBL ARCHIVE 24 March 1999 (1999-03-24), ZHANG Q. ET AL.: "Homo sapiens NG,NG-dimethylarginine dimethylaminohydrolase homolog mRNA, complete cds." XP002287347 Database accession no. AF070667
- SANTA MARIA JOANNE ET AL: "Identification of microbial dimethylarginine dimethylaminohydrolase enzymes" MOLECULAR MICROBIOLOGY, vol. 33, no. 6, September 1999 (1999-09), pages 1278-1279, XP000864652 ISSN: 0950-382X -& DATABASE EMBL 24 May 1999 (1999-05-24), OLIVER K. ET AL.: "Streptomyces coelicolor cosmid 5F2A" XP002288106 Database accession no. AL049587
- TRAN CAM T L ET AL: "The DDAH/ADMA/NOS pathway." ATHEROSCLEROSIS SUPPLEMENTS, vol. 4, no. 4, December 2003 (2003-12), pages 33-40, XP002287345 ISSN: 1567-5688
- KIMOTO M. ET AL.: "Purification, cDNA cloning and expression of human NG,NG-dimethylarginine dimethylaminohydrolase" EUROPEAN JOURNAL OF BIOCHEMISTRY, vol. 258, no. 2, 1 December 1998 (1998-12-01), pages 863-868, XP000864661
- KIMOTO M. ET AL.: "Detection of NG,NG-dimethylarginine dimethylaminohydrolase in human tissues using a monoclonal antibody" JOURNAL OF BIOCHEMISTRY, vol. 117, no. 2, February 1995 (1995-02), pages 237-238, XP000914710
- KIMOTO M. ET AL.: "Cloning and sequencing of a cDNA encoding NG,NG-dimethylarginine dimethylaminohydrolase from rat kidney" BIOCHIMICA ET BIOPHYSICA ACTA, vol. 1337, no. 1, 4 January 1997 (1997-01-04), pages 6-10, XP000864651
- LEIPER J.M. ET AL.: "Identification of two human dimethylarginine dimethylaminohydrolases with distinct distribution and homology with microbial arginine deiminases" BIOCHEMICAL JOURNAL, vol. 343, 1 October 1999 (1999-10-01), pages 209-214, XP002140903

## Description

### Technical field of the invention

This invention relates to methods of screening for compounds which specifically regulate different isoforms of dimethylarginine dimethylaminohydrolase.

### Background of the invention

Arginine residues in proteins are methylated by a family of Protein arginine N-methyltransferases (PRMTs). These enzymes catalyze the methylation of guanidino nitrogens of arginine to produce N^{G} monomethyl-L-arginine (L-NMMA), N^{G}N'^{G} dimethyl-L-arginine (asymmetric dimethylarginine; ADMA) and N^{G}N^{G} dimethylarginine (symmetric dimethylarginine; SDMA). Proteolysis of proteins containing these residues releases free methylarginines. Although the biological role of methylarginine residues is unclear, free L-NMMA and ADMA, but not SDMA, are inhibitors of all three isoforms of nitric oxide synthase (NOS)and might alter NOS activity in health or disease.

Free methylarginines are found in cell cytosol, plasma and tissues and their concentrations differ between tissues and between regions within a single tissue or organ. Elevated concentrations of ADMA have been detected in endothelial cells repopulating blood vessels damaged by balloon injury, in the plasma of patients or experimental animals with hyperlipidaemia, renal failure or athersclerosis, and in patients with schizophrenia or multiple sclerosis. Altered biosynthesis of nitric oxide (NO) has been implicated in the pathogenesis of all of these conditions and it is possible that the accumulation of endogenous ADMA underlies the inhibition of NO generation.

The production of methylarginines is probably an obligatory step in protein turnover, and rates of production may show tissue specific and temporal variations. However, L-NMMA and ADMA, but not SDMA, are actively metabolised to citrulline and methylamines by the action of dimethylarginine dimethylaminohydrolase (DDAH). Certain tissues which express NOSs also appear to express DDAH. Pharmacological inhibition of DDAH increases the concentration of ADMA in endothelial cells and inhibits NO-mediated endothelium-dependent relaxation of blood vessels. These observations suggest that DDAH activity ensures that the local concentration of ADMA does not normally rise sufficiently to affect NO generation, and that changes in DDAH activity could actively alter NOS activity.

### Summary of the invention

The present invention is based on our finding that humans express two functionally active methylarginases, which we have called DDAHI and DDAHII. We have cloned the polynucleotides that encode DDAHI and DDAHII isoforms and have studied the expression patterns of these two methylarginases via RNA blotting. These experiments revealed that DDAHI has a tissue distribution in humans which is similar to that of the neuronal isoform of nitric oxide synthase (nNOS), whilst DDAHII is highly expressed in vascular tissues which also express endothelial (eNOS).

Furthermore, we have shown that DDAHII is expressed in immune tissues and is located to chromosome 6p21.3. That chromosomal locus has been implicated in susceptibility to several diseases, including rheumatoid arthritis and insulin-dependent diabetes mellitus (IDDM) and the altered production of nitric oxide has been implicated in the pathology of both of those diseases.

Our data provide evidence that methylarginine concentration is actively regulated in cells that express NOS and further, suggest that there is a mechanism of regulation of NOS whereby different isoforms of NOS are specifically regulated as methylarginine concentrations are modulated by the action of specific DDAH enzymes.

DDAHI and DDAHII therefore provide new targets for the isolation of substances which can specifically modulate the activity of particular NOS isoforms or other arginine utilising enzymes through specific interaction with particular DDAH isoforms. Such substances may be useful in the treatment of diseases in which abnormal levels of methylarginines and/or nitric oxide are implicated.

Furthermore, we have found that the human DDAHI and DDAHII share significant homology with bacterial arginine deiminases. Arginine deiminases have only been described in prokaryotic organisms and the primitive eukaryote *Giardia intestinalis*. Arginine deiminases catalyse the hydrolysis of arginine to ammonia and citrulline in a reaction that closely resembles the hydrolysis of methylarginine to methylamine and citrulline catalysed by DDAHI.

We have isolated DDAHI sequences from three species of bacteria and an arginine deiminase sequence from *P. aeruginosa.* The enzymes encoded by these sequences can be expressed at high levels and large quantities of the expressed enzyme can be recovered. Thus we have identified an excellent source of enzymes which can be used to identify compounds capable of modulating the activity of DDAH enzymes.

According to the present invention there is thus provided a polynucleotide which:
(a) encodes a polypeptide that has methylarginase activity, which polynucleotide is selected from:
   (1) the coding sequence of SEQ ID NO: 3; and
   (2) a sequence that is degenerate as a result of the genetic code with respect to a sequence defined in (1); or
(b) is a sequence complementary to a polynucleotide defined in (a).

The invention also provides:
- a polypeptide which has methylarginase activity and which comprises the sequence set out in SEQ ID NO: 4.
- a vector incorporating a polynucleotide of the invention;
- a cell harbouring a vector of the invention;
- an antibody capable of binding a polypeptide which has methylarginase activity and which comprises the sequence set out in SEQ ID NO: 4, wherein the antibody is able to discriminate between a DDAHI polypeptide and a DDAHII polypeptide;
- a transgenic non-human animal which is not capable of expressing an active form of a dimethylarginine dimethylaminohydrolase II (DDAHII), wherein, in the transgenic animal, polynucleotide sequence from the DDAHII gene locus has been deleted or replaced with polynucleotide sequence from another locus or another organism or in which the coding sequence of the DDAHII gene has been altered such that the expressed polypeptide shows no methylarginase activity, and wherein said non-human animal is a mammal.
- a process for the preparation of a polypeptide which has methylarginase activity, which process comprises cultivating a host cell harbouring an expression vector of the invention under conditions to provide for expression of the said polypeptide, and recovering the expressed polypeptide; and
- a method for identifying a modulator of methylarginase activity and/or expression, comprising:
   (i) contacting a polynucleotide of the invention, a polypeptide of the invention, a vector of the invention or a cell of the invention and a test substance under conditions that would permit methylarginase activity in the absence of the test substance; and
   (ii) determining thereby whether the said substance modulates the activity and/or expression of methylarginase.

### Brief description of the drawings

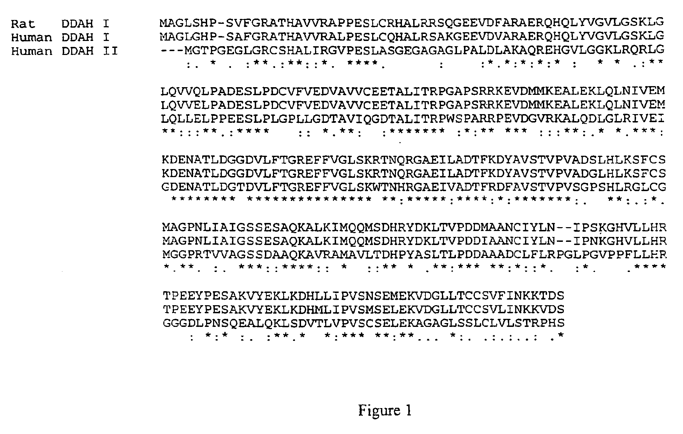

Figure 1 shows an amino acid alignment of rat and human DDAHI with human DDAHII. The derived amino acid sequences of human and rat DDAHI and human DDAHII were aligned using the clustal programme. Amino acid identities are indicated (*), highly conservative substitutions (:) and conservative substitutions (.).

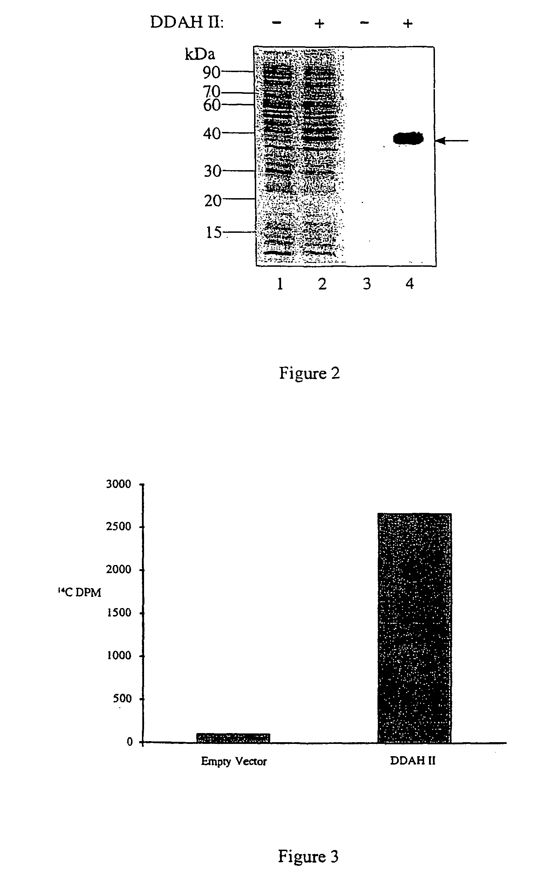

Figure 2 shows recombinant expression of human DDAH II. Aliquots of *E. coli* transfected with either empty vector (lanes 1 and 3) or vector containing human DDAH II cDNA (lanes 2 and 4) were resolved on 15% SDS-PAGE gels. Gels were either stained for total protein with coomassie blue (lanes 1 and 2) or processed for western blotting (lanes 3 and 4) as described under Experimental Procedures. The filled arrow indicates the ∼40kDa recombinant protein that is specifically recognised by the anti-PentaHis antibody. The migration of molecular weight markers is indicated.
Figure 3 shows DDAH activity of recombinant DDAHI II. Aliquots of cell lysates of *E. coli* transfected with either empty vector or vector containing human DDAH II cDNA were assayed for DDAH activity as described under Experimental Procedures. Assays were performed in triplicate and the data is expressed as the average of the three replicates after subtraction of background. The data presented is the result of one representative experiment. Similar results were obtained in four independent experiments. The data shown represent the hydrolyses of ∼1mmol L-NMMA hr⁻¹ by *E. coli* lysates containing recombinant DDAHI II. Under the same conditions, a 30% rat liver homogenate hydrolysed ∼18mmol L-NMMA hr-1

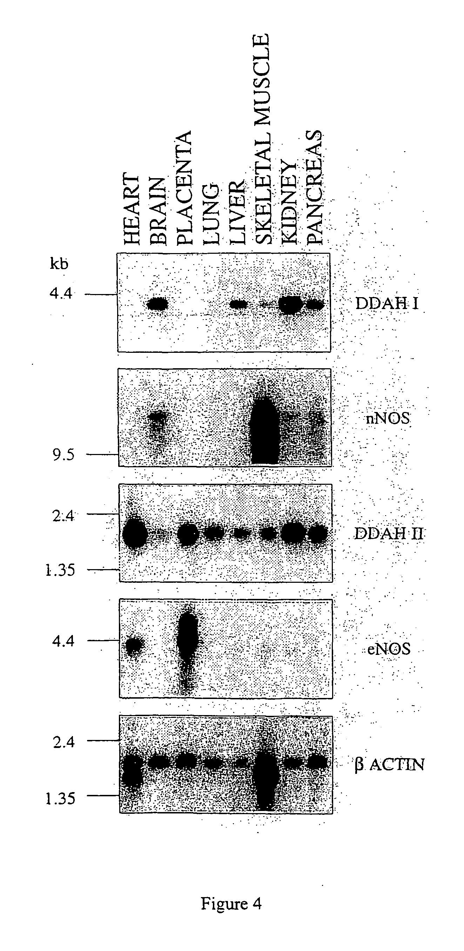

Figure 4 shows tissue distribution of human DDAHI and NOS isoforms. Labelled probes specific for human DDAH I, DDAH II, neuronal NOS, endothelial NOS and b-actin were sequentially hybridized to a commercially availible multiple-tissue northern blot. The migration of molecular weight markers is indicated.

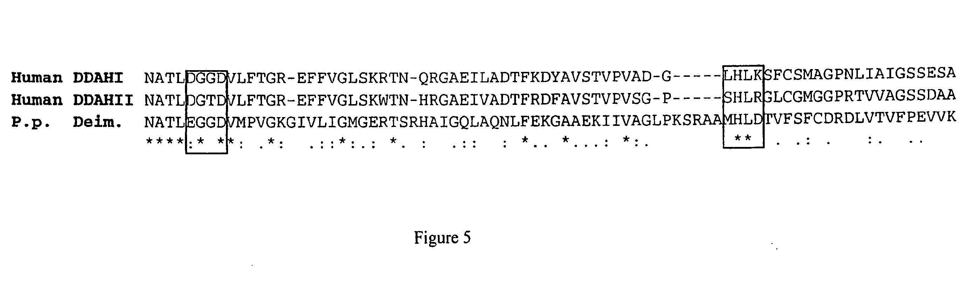

Figure 5 shows aliment of human DDAHI and II with *Pseudomonas* Arginine Deiminase. The derived amino acid of human DDAHI and II were aligned with the amino acid sequence of Pseudomonas X arginine deiminase. Amino acid identities are indicated (*), highly conservative substitutions (:) and conservative substitutions (.). Boxed regions indicated motifs highly conserved between arginine deiminases.

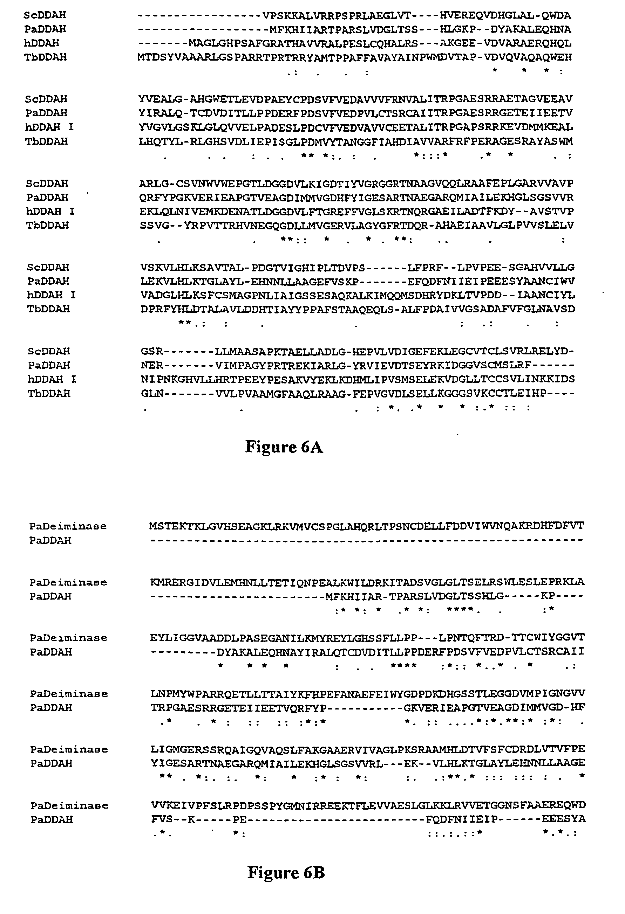

Figure 6A shows the alignment using Clustal W of human DDAH I and DDAHs from *S.coelicolor, P.aeruginosa* and *M.tuberculosis*. Identical amino acids are indicated by (*), highly conserved amino acid substitutions by (:) and conserved amino acid substitutions by (.).
   *S. coelicolor* DDAH is encoded by residues 33784 to 33011 of cosmid St4C6. The sequence does not have an individual accession number. *P. aeruginosa* DDAHI sequence is contained within a contiguous genomic DNA sequence (contig 1281). Again, the sequence does not have an individual accession number. *M. tuberculosis* DDAH has been deposited under accession number DDAH Z797022.
Figure 6B shows a similar alignment using ClustalW of *P. aeruginosa* DDAH and arginine deiminase.
Figure 7 shows enzymatic activity of ScDDAH and PaDDAH. The effect of 10mM ADMA and SDMA on recombinant ScDDAH and paDDAH was studied using the assay conditions described in the material and methods section below. Assays were carried out in triplicate on aliquots of cell lysates containing empty vector, scDDAH cDNA or paDDAH cDNA and data was expressed as a mean of the total number of replicates after subtraction of background. The results shown are the mean of three independent experiments.

### Detailed Description of the Invention

### Polynucleotides

The invention provides a polynucleotide which:
(a) encodes a polypeptide that has methylarginase activity, which polynucleotide is selected from:
   (1) the coding sequence of SEQ ID NO: 3; and
   (2) a sequence that is degenerate as a result of the genetic code with respect to a nucleic sequence defined in (1); or
(b) is a sequence complementary to a polynucleotide defined in (a).

SEQ ID NOS: 1, 3, 5, 7, 9 and 11 set out the sequences of human DDAHI, DDAHII, *S. coelicolor* DDAH, *P. aeruginosa* DDAH, *P. aeruginosa* arginine deiminase and *M. tuberculosis* DDAH respectively.

Polynucleotides of the invention also include variants of the coding sequence of SEQ ID NO: 3 which can function as methylarginases. Such variants thus have the ability to catalyze the production of citrulline from methylarginines. Typically a polynucleotide of the invention comprises a contiguous sequence of nucleotides which is capable of hybridizing under selective conditions to the complement of the coding sequence of SEQ ID NO: 3.

A polynucleotide of the invention and the complement of the coding sequence of SEQ ID NO: 3 can hydridize at a level significantly above background. Background hybridization may occur, for example, because of other cDNAs present in a cDNA library. The signal level generated by the interaction between a polynucleotide of the invention and the complement of the coding sequence of SEQ ID NO: 3 is typically at least 10 fold, preferably at least 100 fold, as intense as interactions between other polynucleotides and the coding sequence of SEQ ID NO: 3. The intensity of interaction may be measured, for example, by radiolabelling the probe, e.g. with ³²P. Selective hybridisation may typically be achieved using conditions of low stringency (0.03M sodium chloride and 0.03M sodium citrate at about 40°C), medium stringency (for example, 0.03M sodium chloride and 0.03M sodium citrate at about 50°C) or high stringency (for example, 0.03M sodium chloride and 0.03M sodium citrate at about 60°C).

A nucleotide sequence which is capable of selectively hybridizing to the complement of the DNA coding sequence of SEQ ID NOS: 3 will generally have at least 50%, at least 70%, at least 80%, at least 90%, at least 95%, at least 98% or at least 99% sequence identity to the coding sequence of SEQ ID NO: 1, 3, 5, 7, 9 or 11 over a region of at least 20, preferably at least 30, for instance at least 40, at least 60, more preferably at least 100 contiguous nucleotides or most preferably over the full length of SEQ ID NO: 3.

Any combination of the above mentioned degrees of sequence identity and minimum sizes may be used to define polynucleotides of the invention, with the more stringent combinations (i.e. higher sequence identity over longer lengths) being preferred. Thus, for example a polynucleotide which has at least 90% sequence identity over 25, preferably over 30 nucleotides forms one aspect of the invention, as does a polynucleotide which has at least 95% sequence identity over 40 nucleotides.

The coding sequence of SEQ ID NO: 3 may be modified by nucleotide substitutions, for example from 1, 2 or 3 to 10, 25, 50 or 100 substitutions. The polynucleotide of SEQ ID NO: 3 may alternatively or additionally be modified by one or more insertions and/or deletions and/or by an extension at either or both ends. The modified polynucleotide generally encodes a polypeptide which has methylarginase activity. Degenerate substitutions may be made and/or substitutions may be made which would result in a conservative amino acid substitution when the modified sequence is translated, for example as shown in the Table below.

Polynucleotides of the invention may comprise DNA or RNA. They may also be polynucleotides which include within them synthetic or modified nucleotides. A number of different types of modification to polynucleotide are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or lifespan of polynucleotides of the invention.

Polynucleotides of the invention may be used as a primer, e.g. a PCR primer, a primer for an alternative amplification reaction, a probe e.g. labeled with a revealing label by conventional means using radioactive or non-radioactive labels, or the polynucleotides may be cloned into vectors.

Such primers, probes and other fragments will preferably be at least 10, preferably at least 15 or at least 20, for example at least 25, at least 30 or at least 40 nucleotides in length. They will typically be up to 40, 50, 60, 70, 100 or 150 nucleotides in length. Probes and fragments can be longer than 150 nucleotides in length, for example up to 200, 300, 400, 500, 500, 700 nucleotides in length, or even up to a few nucleotides, such as five or ten nucleotides, short of the coding sequence of SEQ ID NO: 3.

Polynucleotides such as a DNA polynucleotide and primers according to the invention may be produced recombinantly, synthetically, or by any means available to those of skill in the art. They may also be cloned by standard techniques. The polynucleotides are typically provided in isolated and/or purified form.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using PCR (polymerase chain reaction) cloning techniques. This will involve making a pair of primers (e.g. of about 15-30 nucleotides) to a region of the G14 gene which' it is desired to clone, bringing the primers into contact with mRNA or cDNA obtained from a human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Such techniques may be used to obtain all or part of the DDAHI and DDAHII genes described herein. Genomic clones corresponding to the cDNA of SEQ ID NO: 3 containing, for example, introns and promoter regions are also aspects of the invention and may also be produced using recombinant means, for example using PCR (polymerase chain reaction) cloning techniques, starting with genomic DNA from for example a bacterial, an animal or a human cell.

Although in general the techniques mentioned herein are well known in the art, reference may be made in particular to Sambrook et al, 1989, Molecular Cloning: a laboratory manual.

Polynucleotides which do not have 100% sequence identity to the sequence of SEQ ID NO: 3 but fall within the scope of the invention can be obtained in a number of ways:

1. Other human allelic variants of the human DDAHII sequences given in SEQ ID NO: 3 may be obtained for example by probing genomic DNA libraries made from a range of individuals, for example individuals from different populations, or individuals with different types of disorder related to aberrant NO metabolism, using probes as described above.

In addition, homologues of SEQ ID NO: 3 may be obtained from other animals particularly mammals (for example mice and rabbits) or fish (for example Fugu) or insects (for example *D. melanogaster*)or other invertebrates (for example *C. elegrms*), plants (for example *A. thaliana*), bacteria and yeasts and such homologues and fragments thereof in general will be capable of selectively hybridising to the coding sequence of SEQ ID NO: 3 or its complement. Such sequences may be obtained by probing cDNA or genomic libraries from dividing cells or tissues or other animal species with probes as described above. Degenerate probes can be prepared by means known in the art to take into account the possibility of degenerate variation between the DNA sequence of SEQ ID NO: 3 and the sequences being probed for under the selective hybridization conditions given above.

2. Allelic variants and species homologues may also be obtained using degenerate PCR which will use primers designed to target sequences within the variants and homologues encoding likely conserved amino acid sequences. Likely conserved sequences can be predicted from aligning the ammo acid sequences of the invention. The primers will contain one or more degenerate positions and will be used at stringency conditions lower than those used for cloning sequences with single sequence primers against known sequences.

3. Alternatively, polynucleotides may be obtained by site directed mutagenesis of SEQ ID NO: 3 or allelic variants thereof. This may be useful where, for example, silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

The invention further provides double stranded polynucleotides comprising a polynucleotide of the invention and its complement.

Polynucleotides, probes or primers of the invention may carry a revealing label. Suitable labels include radioisotopes such as ³²P or ³⁵S, enzyme labels, or other protein labels such as biotin. Such labels may be added to polynucleotides, probes or primers of the invention and may be detected using techniques known *per se*.

### Polypeptides

A polypeptide of the invention comprises the amino acid sequence set out in SEQ ID NO: 4 or a substantially homologous sequence, or a fragment of either said sequence and has methylarginase activity. In general, the naturally occurring amino acid sequence shown in SEQ ID NO: 4 is preferred.

SEQ ID NOS: 2, 4, 6, 8, 10 and 12 set out the amino acid sequences of human DDAHII, human DDAHII, *S. coelicolor* DDAH, *P.aeruginosa* DDAH, P.aeruginosa arginine deiminase and *M.tuberculosis* DDAH respectively

In particular, a polypeptide of the invention may comprise:
(a) the polypeptide sequence of SEQ ID NO: 4;
(b) an allelic variant or species homologue thereof; or
(c) a protein with at least 70, at least 80, at least 90, at least 95, at least 98 or at least 99% sequence identity to (a) or (b).

An allelic variant will be a variant which will occur naturally, for example, in a human, bacterium or yeast and which will function in a substantially similar manner to the protein of SEQ ID NO: 4 for example it acts as a methylarginase. Similarly, a species homologue of the protein will be the equivalent protein which occurs naturally in another species and which can function as a methylarginase.

Allelic variants and species homologues can be obtained by following the procedures described herein for the production of the polypeptides of SEQ ID NO: 2, 4, 6, 8, 10 or 12 and performing such procedures on a suitable cell source e.g. a human or bacterium cell. It will also be possible to use a probe as defined above to probe libraries made from human or bacterial cells in order to obtain clones encoding the allelic or species variants. The clones can be manipulated by conventional techniques to generate a polypeptide of the invention which can then be produced by recombinant or synthetic techniques known *per se*.

A polypeptide of the invention preferably has at least 60% sequence identity to the protein of SEQ ID NO: 4, more preferably at least 70%, at least 80%, at least 90%, at least 95%, at least 97% or at least 99% sequence identity thereto over a region of at least 20, preferably at least 30, for instance at least 40, at least 60, at least 100 contiguous amino acids or over over the full length of SEQ ID NO: 4.

The sequence of the polypeptide of SEQ ID NO: 4 and of allelic variants and species homologues can thus be modified to provide polypeptides of the invention. Amino acid substitutions may be made, for example from 1, 2 or 3 to 10, 20 or 30 substitutions. The modified polypeptide generally retains activity as a methylarginase, as defined herein. Conservative substitutions may be made, for example according to the following Table. Amino acids in the same block in the second column and preferably in the same line in the third column may be substituted for each other.

| | | |
|---|---|---|
| ALIPHATIC | Non-polar | G A P |
| | | I L V |
| | Polar-uncharged | C S T M |
| | | N Q |
| | Polar-charged | D E |
| | | K R |
| AROMATIC | | H F W Y |

Polypeptides of the invention also include fragments of the above-mentioned full length polypeptides and variants thereof, including fragments of the sequence set out in SEQ ID NO: 4. Such fragments typically retain activity as a methylarginase.

Other preferred fragments include those which include an epitope. Suitable fragments will be at least 5, e.g, at least 10, at least 12, at least 15 or at least 20 amino acids in size. Epitope fragments may typically be up to 50, 60, 70, 80, 100, 150 or 200 amino acids in size. Polypeptide fragments of the polypeptides of SEQ ID NO: 3, and allelic and species variants thereof may contain one or more (e.g. 1, 2, 3 or 5 to 10, 20 or 30) substitutions, deletions or insertions, including conservative substitutions. Epitopes may be determined by techniques such as peptide scanning techniques already known in the art. These fragments will be useful for obtaining antibodies to polypeptides of the invention.

Polypeptides of the invention may be in a substantially isolated form. It will be understood that the polypeptide may be mixed with carriers or diluents which will not interfere with the intended purpose of the polypeptide and still be regarded as substantially isolated. A polypeptide of the invention may also be in a substantially, purified form, in which case it will generally comprise the polypeptide in a preparation in which more than 50%, e.g. more than 80%, 90%, 95% or 99%, by weight of the polypeptide in the preparation is a polypeptide of the invention.

Polypeptides of the invention may be chemically modified, e.g. post-translationally modified. For example, they may be glycosylated or comprise modified amino acid residues. They may also be modified by the addition of Histidine residues to assist their purification or by the addition of a signal sequence to promote their secretion from a cell. Such modified polypeptides and proteins fall within the scope of the term "polypeptide" of the invention.

Polypeptides of the invention may be modified for example by the addition of Histidine residues or a T7 tag to assist their identification or purification or by the addition of a signal sequence to promoter their secretion from a cell where the polypeptide does not naturally contain such a sequence.

### Vectors

Polynucleotides of the invention can be incorporated into a recombinant replicable vector. The vector may be used to replicate the nucleic acid in a compatible host cell. Thus, in a further embodiment, the invention provides a method of making polypeptides of the invention by introducing a polynucleotide of the invention into a replicable vector, introducing the vector into a compatible host cell, and growing the host cell under conditions which bring about replication of the vector. The vector may be recovered from the host cell.

Polynucleotides according to the invention may also be inserted into the vectors described above in an antisense orientation in order to provide for the production of antisense RNA. Antisense RNA or other antisense polynucleotides may also be produced by synthetic means. Such antisense polynucleotides may be used in a method of controlling the levels of methylarginases or their variants or species homologues.

Preferably, a polynucleotide of the invention in a vector is operably linked to a control sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence, such as a promoter, "operably linked" to a coding sequence is positioned in such a way that expression of the coding sequence is achieved under conditions compatible with the regulatory sequence.

Vectors of the invention may be transformed into a suitable host cell as described above to provide for expression of a polypeptide of the invention. Thus, in a further aspect the invention provides a process for preparing a polypeptide according to the invention which comprises cultivating a host cell transformed or transfected with an expression vector encoding the polypeptide, and recovering the expressed polypeptide.

The vectors may be for example, plasmid, virus or phage vectors provided with a origin of replication, optionally a promoter for the expression of the said polynucleotide and optionally a regulator of the promoter. The vectors may contain one or more selectable marker genes, for example an ampicillin residence gene in the case of a bacterial plasmid or a resistance gene for a fungal vector. Vectors may be used *in vitro*, for example for the production ofRNA or used to transfect or transform a host cell, for example, *E. coli*. The vectors may also be adapted to be used *in vivo*, for example in a method of gene therapy.

A further embodiment of the invention provides host cells transformed or transfected with the vectors for the replication and/or expression of polynucleotides of the invention. The cells will be chosen to be compatible with the said vector and may for example baterial (eg. *E. coli*), yeast, insect or mammalian.

Promoters and other expression regulation signals may be selected to be compatible with the host cell for which expression is designed. For example, yeast promoters include *S. cerevisiae* GAL4 and ADH promoters, *S. pombe nmt*1 and *adh* promoter. Mammalian promoters include the metallothionein promoter which can be induced in response to heavy metals such as cadmium. Viral promoters such as the SV40 large T antigen promoter or adenovirus promoters may also be used. All these promoters are readily available in the art.

Mammalian promoters, such as β-actin promoters, may be used. Tissue-specific promoters, in particular endothelial or neuronal cell specific promoters (for example the DDAHI and DDAHII promoters), are especially preferred. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, adenovirus, HSV promoters (such as the HSV IE promoters), or HPV promoters, particularly the HPV upstream regulatory region (URR). Viral promoters are readily available in the art.

The vector may further include sequences flanking the polynucleotide giving rise to antisense RNA which comprise sequences homologous to eukaryotic genomic sequences, preferably mammalian genomic sequences, or viral genomic sequences. This will allow the introduction of the polynucleotides of the invention into the genome of eukaryotic cells or viruses by homologous recombination. In particular, a plasmid vector comprising the expression cassette flanked by viral sequences can be used to prepare a viral vector suitable for delivering the polynucleotides of the invention to a mammalian cell. Other examples of suitable viral vectors include herpes simplex viral vectors (for example as disclosed in WO 98/04726 and WO 98/30707) and retroviruses, including lentiviruses, adenoviruses, adeno-associated viruses and HPV viruses (such as HPV-16 or HPV-18). Gene transfer techniques using these viruses are known to those skilled in the art. Retrovirus vectors for example may be used to stably integrate the polynucleotide giving rise to the antisense RNA into the host genome. Replication-defective adenovirus vectors by contrast remain episomal and therefore allow transient expression.

### Antibodies

The invention also provides monoclonal or polyclonal antibodies to polypeptides encoded by polynucleotides of the invention, to polypeptides of the invention and to fragments thereof The invention further provides a process for the production of monoclonal or polyclonal antibodies of the invention.

Antibodies of the invention may be antibodies to human polypeptides or fragments thereof. Preferred antibodies are those which are able to discriminate between a DDAHI polypeptide or fragment thereof and a DDAHII polypeptide or fragment thereof, and in particular between the human DDAHI and II polypeptides and fragments thereof.

For the purposes of this invention, the term "antibody", unless specified to the contrary, includes fragments of whole antibodies which maintain their binding activity for a polypeptide encoded by a polynucleotide of the invention, a polypeptide of the invention or a fragment thereof. Such fragments include Fv, F(ab') and F(ab')₂ fragments, as well as single chain antibodies. Furthermore, the antibodies and fragment thereof may be chimeric antibodies, CDR-grafted antibodies or humanised antibodies.

Antibodies of the invention may be used, *inter alia*, in a method for detecting polypeptides of the invention present in a biological sample which method comprises
(a) providing an antibody of the invention;
(b) incubating a biological sample with said antibody under conditions which allow for the formation of an antibody-antigen complex; and
(c) determining whether antibody-antigen complex comprising said antibody is forme.

A sample may be for example a tissue extract Antibodies of the invention may be bound to a solid support and/or packaged into kits in a suitable container along with suitable reagents, controls, instructions, etc.

Antibodies of the invention can be produced by any suitable method. Means for preparing and characterising antibodies are well known in the art, see for example Harlow and Lane (1988) "Antibodies: A Laboratory Manual", cold Spring Harbor Laboratory Press, Cold Spring Harbor, NY. For example, an antibody may be produced by raising antibody in a host animal against the whole polypeptide or a fragment thereof, for example an antigenic epitope thereof, herein after the "immunogen".

A method for producing a polyclonal antibody comprises immunising a suitable host animal, for example an experimental animal, with the immunogen and isolating immunoglobulins from the serum. The animal may therefore be inoculated with the immunogen, blood subsequently removed from the animal and the IgG fraction purified.

A method for producing a monoclonal antibody comprises immortalising cells which produce the desired antibody. Hybridoma cells may be produced by fusing spleen cells from an inoculated experimental animal with tumour cells (Kohler and Milstein (1975) Nature 256, 495-497).

An immortalized cell producing the desired antibody may be selected by a conventional procedure. The hybridomas may be grown in culture or injected intraperitoneally for formation of ascites fluid or into the blood stream of an allogenic host or immunocompromised host. Human antibody may be prepared by *in vitro* immunisation of human lymphocytes, followed by transformation of the lymphocytes with Epstein-Barr virus.

For the production of both monoclonal and polyclonal antibodies, the experimental animal is suitably a goat, rabbit, rat or mouse. If desired, the immunogen may be administered as a conjugate in which the immunogen is coupled, for example via a side chain of one of the amino acid residues, to a suitable carrier. The carrier molecule is typically a physiologically acceptable carrier. The antibody obtained may be isolated and, if desired, purified.

### Animals

The invention provides a non-human animal which is not capable of expressing or is not capable of expressing an active form of one or more isoforms of methylarginase. Preferably the methylarginase isoform is a DDAHI and/or a DDAH II. Typically, the wild-type of the non-human animal will be capable of expressing an active form of the methylarginase which the non-human animal of the invention cannot.

An animal which is not capable of expressing one or more isoforms of methylarginase is one which shows substantially no detectable expression of at least one methylarginase mRNA. An animal which is not capable of expressing an active form of one or more isoforms of methylarginase is one which expresses at least one methylarginase related polypeptide, which polypeptide shows substantially no methylarginase activity.

An animal of the invention may be one in which polynucleotide sequence from a methylarginase encoding gene locus has been deleted or replaced with polynucleotide sequences from another locus or from another organism. Thus, substantially no methylarginase mRNA may be expressed from that methylarginase locus. Alternatively, the coding sequence of a methylarginase gene may have been altered such that the expressed polypeptide shows substantially no methylarginase activity.

Typically a non-human animal of the invention is a so-called "knock-out animal". The term "knock-out animal" is well known to those skilled in the art. Typically, a non-human animal of the invention, for example a knock-out animal, will be a transgenic animal.

A knock-out animal can be produced according to any suitable method. In general, a polynucleotide construct is produced comprising a marker gene, for example, flanked by genomic sequences. Those genomic sequences correspond to genomic sequences at the methylarginase encoding gene locus of the animal in question. Thus, if the polynucleotide construct is contacted with the methylarginase encoding gene locus of the animal of interest, homologous recombination events may lead to replacement of the chromosomal sequence bordered by the genomic sequences used in the polynucleotide construct with the marker gene. If the marker gene replaces coding sequence or a regulatory sequence, for example a promoter sequence, gene expression and/or activity may be abolished.

The polynucleotide construct is typically transferred into a fertilized egg by pronuclear microinjection so that the contacting described above can occur. Alternative approaches may be used for example, embryonic stem cells or retroviral mediated gene transfer into germ lines. Whichever approach is taken, transgenic animals are then generated. For example, microinjected eggs may be implanted into a host female and the progeny may be screened for the expression of the marker gene. The founder animals that are obtained may be bred.

A non-human animal of the invention is a mammal, preferably a mouse. Preferred non-human animals are mice. Preferred animals are thus mice in which all or part of the DDAHII gene locus has been deleted or replaced for example, ie. DDAHII knock-out mice.

The invention also provides a cell or cell-line derived from a non-human animal of the invention.

The transgenic technology described above is of course equally applicable to the production of non-human animals which over-express one or more isoforms of methylarginase, or express one or more isoforms of methylarginase which have unusually high activities. In such cases the polynucleotide construct used does not replace an endogenous portion of a methylarginase gene with a marker gene. Instead, an endogenous methylarginase gene may be replaced with a polynucleotide construct comprising a promoter, for example one which drives high levels of expression, operably lined to a methylarginase coding sequence. Alternatively, the construct may comprise a methylarginase promoter sequence operably linked to a reporter gene. It is also possible to produce constructs which do not replace endogenous methylarginase sequences. Use of such constructs will result in animals which contain endogenous methylarginase sequences and the sequences insert by the construct

The non-human animals described above may be used in the screening assays described below for the identification of modulators of methylarginase activity and/or expression.

### Assays

The invention provides a method for identifying a modulator of methylarginase activity and/or expression, comprising:
(i) contacting a polynucleotide according to the invention, a polypeptide according to the invention, a vector according to the invention or a cell according to the invention and a test substance under conditions that would permit methylarginase activity in the absence of the test substance; and
(ii) determining thereby whether the said substance modulates the activity and/or expression of methylarginase.

Any suitable assay formal may be used for identifying a modulator of methylarginase activity and/or expression.

In the case of using a polynucleotide or vector of the invention, the assay will typically be carried out on a cell harbouring the polynucleotide or vector or on a cell extract comprising the polynucleotide or vector. The cell or cell extract will typically allow transcription and translation of the polynucleotide or vector in the absence of a test substance.

A typical assay is as follows:
- a defined number of cells harbouring a polynucleotide or vector of the invention are inoculated in growth medium into the wells of a plastics micro-titre plate in the presence of a substance to be tested.
- the micro-titre plates are covered and incubated at an appropriate temperature (eg. 37°C for *E. coli*) in the dark.
- samples are withdrawn at regular time intervals and assayed for methylarginase activity, as described in the Examples.
- parallel control experiments can be carried out, in which the substance to be tested is omitted.

Also, as a control, the samples may be assayed for any other enzyme to exclude the possibility that the test substances is a general inhibitor of gene expression or enzyme activity.

The assay may also be carried out using a polypeptide of the invention, in which any suitable format may be used for identifying a modulator of methylarginase activity. Most preferably such an assay would be carried out in a single well of a plastics microtitre plate, so that high through-put screening for methylarginase activity modulators may be carried out. In practice, the enzyme reaction is commenced by addition of a methylarginase or a substrate for methylarginase. An assay for a methylarginase modulator may therefore be initiated by providing a medium, containing a test substance and one of a methylarginase and a methylarginase substrate. As a control, the progress of the assay can be followed in the absence of the test substance.

Also the substance tested may be tested with any other known polypeptide/enzyme to exclude the possibility that the test substance is a general inhibitor of enzyme activity.

Suitable methylarginases for the assay can be obtained using the recombinant techniques described above. Suitable substrates are those comprising asymmetric methylarginines, for example N^{G}monomethyl-L-arginine (L-NMMA), asymmetric dimethylarginine (ADMA). In addition to the methylarginase and a suitable substrate, the reaction mixture can contain a suitable buffer, suitable cofactors and suitable divalent cations as a cofactor. A suitable buffer includes any suitable biological buffer that can provide buffering capability at a pH conducive to the reaction requirements of the enzyme.

The assay of the invention may be carried out at any temperature at which a methylarginase, in the absence of any inhibitor, is active. Typically, however, the assay will be carried out in the range of from 25°C to 37°C.

Measures of enzymatic activity of methylarginase activity are generally known to those skilled in the art, including equilibrium constants, reaction velocities of the appearance of reaction products or the consumption of reaction substrates, reaction kinetics, thermodynamics of reaction, spectrophotometric analysis of reaction products, detection of labelled reaction components, etc. See, generally, Segel, Biochemical Calculations, 2nd Edition, John Wiley and Sons, New York (1976); Suelter, A Practial Guide to Enzymology, John Eiley and Sons, New York (1985). The preferred method of measuring enzymatic activity is by measuring [¹⁴C]citrulline production after the methylarginase has been incubated with [¹⁴C]L-NMMA or [¹⁴C]ADMA.

Assays can also be carried out using constructs comprising a a methylarginase gene promoter operably linked to a heterologous coding sequence, to identify compounds which modulate expression of methylarginases at the transcriptional level.

A promoter means a transcriptional promoter. Methylarginase gene promoters can be isolated via methods known to those skilled in the art and as described above. The term "heterologous" indicates that the coding sequence is not operably linked to the promoter in nature; the coding sequence is generally from a different organism to the promoter.

The promoter sequence may be fused directly to a coding sequence or via a linker. The linker sequence may comprise an intron. Excluding the length of any intron sequence, the linker may be composed of up to 45 bases. The linker sequence may comprise a sequence having enhancer characteristics, to boost expression levels.

Preferably the promoter is operably linked to the coding sequence of a reporter polypeptide. The reporter polypeptide may be, for example, the bacterial polypeptide β-glucuronidase (GUS), green fluoresent protein (GFP), luciferase (luc), chloramphenicol transferase (CAT) or β-galactosidase (lacZ).

Promoter:reporter gene constructs such as those described above can be incorporated into a recombinant replicable vector. The vector may be used to replicate the nucleic acid construct in a compatible host cell. The vectors may be, for example, plasmid, virus or phage vectors provided with an origin of replication. Any host cell may be used in which the promoter is functional, but typically the host cell will be a cell of the species from which the promoter derives. The promoter:reporter gene constructs of the invention may be introduced into host cells using conventional techniques.

Thus the invention provides a method for identifying a modulator of methylarginase expression. Typically a promoter:reporter polypeptide construct or a cell harbouring that construct will be contacted with a test substance under conditions that would permit the expression of the reporter polypeptide in the absence of the test substance.

Any reporter polypeptide may be used, but typically GUS or GFP are used. GUS is assayed by measuring the hydrolysis of a suitable substrate, for example 5-bromo-4-chloro-3-indolyl-β-D-glucoronic acid (X-gluc)or 4-methylumbelliferyl-β-glucuronide (MUG). The hydrolysis of MUG yields a product which can be measured fluorometrically. GFP is quantified by measuring fluorescence at 590nm after excitation at 494nm. These methods are well known to those skilled in the art.

### Methylarginases

A methylarginase encoded by a polynucleotide of the invention or a methylarginase having the amino acid sequence of a polypeptide of the invention may be used in the assays described above. The enzymes may be obtained from extracts. Alternatively, the enzymes may be produced recombinantly, from, for example, bacteria, yeast or higher eukaryotic cells such as insect cell lines. A preferred methylarginase is human DDAHII having the sequence shown in SEQ ID NO: 4.

Recombinant expression of human DDAHII and bacterial DDAHI enzymes is described in the Examples.

### Test Substances

A substance which modulates the expression or activity of a methylarginase may do so by binding directly to the relevant gene promoter, thus inhibiting or activating transcription of the gene. Inhibition may occur by preventing the initiation or completion of transcription. Activation may occur, for example, by increasing the affinity of the transcription complex for the promoter. Alternatively a modulator may bind to a protein which is associated with the promoter and is required for transcription.

A substance which modulates the activity of a methylarginase may do so by binding to the enzyme. Such binding may result in activation or inhibition of the protein.

Inhibition may occur, for example if the modulator resembles the substrate and binds at the active site of the methylarginase. The substrate is thus prevented from binding to the same active site and the rate of catalysis is reduced by reducing the proportion of enzyme molecules bound to substrate. A modulator which inhibits the activity of a methylarginase may do so by binding to the substrate. The modulator may itself catalyze a reaction of the substrates, so that the substrate is not available to the enzyme. Alternatively, the inhibitor may simply prevent the substrate binding to the enzyme.

Activation may occur, for example, if the modulator increases the affinity of the substrate for the enzyme or *vice versa*. This means that the proportion of enzyme molecules bound to a substrate is increased and the rate of catalysis will thus increase.

Suitable test substances which can be used in the assays described above include antibody products (for example, monoclonal and polyclonal antibodies, single chain antibodies, chimeric antibodies and CDR-grafted antibodies) which are specific for a methylarginase or mimics of a methylarginase. In addition, combinatorial libraries, defined chemical identities, peptide and peptide mimetics, oligonucleotides and natural product libraries, such as display libraries (e.g. phage display libraries) may also be tested. The candidate substances may be chemical compounds.

Batches of the test substances may be used in an initial screen of, for example, ten substances per reaction, and the substances of batches which show inhibition tested individually.

### Modulators

A modulators of methylarginase, for example of a DDAH or an arginine deiminase, expression and/or activity is one which produces a measurable redaction or increase in methylarginase expression and/or activity in the assays described above. Thus, modulators of methylarginase expression and/or activity may be inhibitors or activators of methylarginase expression and/or activity. A modulator of a methylarginase may be a modulator of a DDAHI or a DDAHII.

Preferred inhibitors are those which inhibit methylarginase expression and/or activity by at least 10%, at least 20%, at least 30%, at least 40% at least 50%, at least 60%, at least 70%, at least 80%, at least 90%, at least 95% or at leas 99% at a concentration of the inhibitor of 1µg ml⁻¹, 10µg ml⁻¹, 100µg ml⁻¹, 500µg ml⁻¹, 1mg ml^{-1,} 10mg ml⁻¹, 100mg ml⁻¹.

Preferred activitors are those which activate methylarginase expression and/or activity by at least 10%, at least 25%, at least 50%, at least 100%, at least, 200%, at least 500% or at least 1000% at a concentration of the activators 1 µg ml⁻¹, 10µg ml⁻¹, 100µg ml⁻¹, 500µg ml⁻¹, 1mg ml^{-1,} 10mg ml⁻¹, 100mg ml⁻¹.

The percentage inhibition or activation represents the percentage decrease or increase in expression/activity in a comparison of assays in the presence and absence of the test substance. Any combination of the above mentioned degrees of percentage inhibition or activation and concentration of inhibitor or activator may be used to define an inhibitor or activator, with greater inhibition or activation at lower concentrations being preferred.

Candidate substances which show activity in assays such as those described above can be tested in *in vivo* systems, an animal model. Candidate inhibitors could be tested for their ability to increase ADMA and L-NMMA levels and/or to increase bloom pressure and/or to decrease endothelium-dependent relaxation of blood vessels.

Candidate activators could be tested for their ability to increase nitric oxide generation as assessed by NOₓ measurement and/or to decrease levels of ADMA and L-NMMA. Ultimately such substances would be tested in animal models of the target disease states.

### Therapeutic use

Polynucleotides, peptides, expression vectors and modulators of methylarginase activity and/or expression and modulators of methylarginase activity and/or expression identified by the methods of the invention may be used for the treatment of a condition in which the abnormal metabolism of NO is implicated.

Polynucleotides, peptides, expression vectors and activators of methylarginase activity and/or expression may be used in the treatment of conditions in which reduced NO production is implicated. In particular such conditions as hyperlipidaemia, renal failure, hypertension, restenosis after angioplasty, complications of heart failure, or atherosclerosis and its complications may be treated and patients with schizophrenia, multiple sclerosis or cancer may also be treated.

Modulators which are inhibitors of methylarginase activity and/or expression may be used in the-treatment of conditions in which increased NO production is implicated. In particular conditions such as ischeamia-reperfusion injury of the brain or heart, cancer, lethal hypotension in severe inflammatory conditions such as septic shock or multi-organ failure, or local and systemic inflammatory disorders including arthritis, skin disorders, inflammatory cardiac disease or migraine may be treated.

Alternatively an inhibitor of methylarginase activity and/or expression could be used as a joint therapy together with an inhibitor of NOS activity (for example, a methylarginine). For example, a specific inhibitor of a DDAH isoform could be used with the methylarginine L-NMMA. This approach may radically alter the activity profile of L-NMMA and may result in L-NMMA having an increased inhibitory effect for a specific NOS isoform. Thus, products containing an inhibitor of methylarginase activity and/or expression and a methylarginine as a combined preparation for simultaneous, separate or sequential use in the treatment of ischeamia-reperfusion injury of the brain or heart, cancer, lethal hypotension in severe inflammatory conditions such as septic shock or multi-organ failure, or local and systemic inflammatory disorders including arthritis, skin disorders, inflammatory cardiac disease or migraine.

Inhibitors of methylarginase expression and/or activity may also be used as antimicrobial, for example antibacterial, agents. Thus, inhibitors of microbial, for example bacterial, DDAH and arginine deiminase expression and/or activity are useful as antimicrobial agents. Therefore, an inhibitor of a bacterial methylarginase, for example a DDAH or an arginine deiminase, for use in the treatment of a bacterial infection.

A modulator may be administered in a variety of dosage forms. Thus, they can be administered orally, for example as tablets, troches, lozenges, aqueous or oily suspensions, dispersible powders or granules. The modulators may also be administered parenterally, either subcutaneously, intravenously, intramuscularly, intrasternally, transdermally or by infusion techniques. The modulators may also be administered as suppositories. A physician will be able to determine the required route of administration for each particular patient.

The formulation of a modulator for use in preventing or treating the conditions described above will depend upon factors such as the nature of the exact inhibitor, whether a pharmaceutical or veterinary use is intended, etc. An modulator may be formulated for simultaneous, separate or sequential use.

An modulator is typically formulated for administration in the present invention with a pharmaceutically acceptable carrier or diluent The pharmaceutical carrier or diluent may be, for example, an isotonic solution. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for intravenous administration or infusion may contain as carrier, for example, sterile water or preferably they may be in the form of sterile, aqueous, isotonic saline solutions.

A therapeutically effective amount of a modulator is administered to a patient. The dose of a modulator may be determined according to various parameters, especially according to the substance used; the age, weight and condition of the patient to be treated; the route of administration; and the required regimen. Again, a physician will be able to determine the required route of administration and dosage for any particular patient A typical daily dose is from about 0.1 to 50 mg per kg of body weight, according to the activity of the specific modulator, the age, weight and conditions of the subject to be treated, the type and severity of the degeneration and the frequency and route of administration. Preferably, daily dosage levels are from 5 mg to 2 g.

The expression and/or activity of a particular isoform of NOS may be specifically regulated. Substances which have effects specific for one particular methylarginase isoform, for example a DDAHI or a DDAHII, may be administered non-specifically as they will only modulate the expression or activity of a particular methylarginase and thus the activity of one particular isoform of NOS.

Some substances may, however, have affect more than one isoform of methylarginase. Such modulators may have to be administered to specific sites, if they are required to regulate only one particular isoform of NOS. For example, if a condition requires the regulation of nNOS the modulator will have to be delivered to neurons. This may be achieved, for example, by delivery via a viral strain such as herpes simplex virus. Viral vectors comprising polynucleotides of the invention are described above. The viral vector delivery method may be used in the case of administration of, for example, polynucleotides of the invention.

The polynucleotides and vectors of the invention may be administered directly as a naked nucleic acid construct. Uptake of naked nucleic acid constructs by mammalian cells is enhanced by several known transfection techniques for example those including the use of transfection agents. Example of these agents include cationic agents (for example calcium phosphate and DEAE-dextran) and lipofectants (for example lipofectam^{™} and transfectam^{™}).

Typically, nucleic acid constructs are mixed with the transfection agent to produce a composition. Preferably the naked nucleic acid construct, viral vector comprising the polynucleotide or composition is combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, subcutaneous, or transdermal administration.

The pharmaceutical composition is administered in such a way that the polynucleotide of the invention, viral vector for gene therapy, can be incorporated into cells at an appropriate area. When the polynucleotide of the invention is delivered to cells by a viral vector, the amount of virus administered is in the range of from 10⁶ to 10¹⁰ pfu, preferably from 10⁷ to 10⁹ pfu, more preferably about 10⁸ pfu for adenoviral vectors. When injected, typically 1-2 ml of virus in a pharmaceutically acceptable suitable carrier or diluent is administered. When the polynucleotide of the invention is administered as a naked nucleic acid, the amount of nucleic acid administered is typically in the range of from 1 µg to 10 mg.

Where the polynucleotide giving rise to the product is under the control of an inducible promoter, it may only be necessary to induce gene expression for the duration of the treatment. Once the condition has been treated, the inducer is removed and expression of the polypeptide of the invention ceases. This will clearly have clinical advantages. Such a system may, for example, involve administering the antibiotic tetracycline, to activate gene expression via its effect on the tet repressot/VP16 fusion protein.

The use of tissue-specific promoters will be of assistance in the treatment of disease using the polypeptides, polynucleotide and vectors of the invention. It will be advantageous to be able express therapeutic genes in only the relevant affected cell types, especially where such genes are toxic when expressed in other cell types.

The routes of administration and dosages described above are intended only as a guide since a skilled physician will be able to determine readily the optimum route of administration and dosage for any particular patient and condition.

The invention is illustrated by the following Example:

### Example

### Materials and methods

Unless otherwise indicated, the methods used are standard biochemistry and molecular biology techniques. Examples of suitable methodology textbooks include Sambrook et al., Molecular Cloning, A Laboratory Manual (1989) and Ausubel et al., Current Protocols in Molecular Biology (1995), John Wiley and Sons, Inc.

### Database searching and cDNA cloning

The cDNA sequence of human DDAHI was obtained by a combination of database searching, specific RT-PCR and 5'/3' RACE. The database of expressed sequence tags (dbEST) was searched with the cDNA sequence corresponding to the open reading frame of rat DDAHI (Kimoto, M., Sasakawa, T., Tsuji, H., Miyatake, S., Oka, T., Nio, N. and Ogawa, T., 1997, Biochim. Biophys. Acta 1337, 6-10) using the 'blast' programme. This search identified a single human cDNA sequence that comprised 161bp of human DDAHI cDNA fused downstream of 160bp of unknown sequence. Using this sequence, two human DDAHI specific oilogonucleotide primers HDDAHI.1 and HDDAHI-2 were designed. Human kidney polyA+ RNA was reverse transcribed from an oligo dT primer, following which human DDAHI cDNA was PCR amplified in two. PCR reactions incorporating either HDDAHI.1 and RDDAHI.1 or HDDAHI.2 and RDDAHI.2. In order to determine the sequence of the 5' and 3' ends of the human DDAHI open reading frame 5' and 3' RACE was performed. For 5' RACE human kidney polyA mRNA was reverse transcribed using primer HDDAHI.3. Following reverse transcription, RNA was digested with RNAse H and cDNA purified using a HighPure DNA purification kit (Boehringer). Purified cDNA was polyA tailed by incubation with terminal transferase in the presence of dATP. Tailed cDNA was used directly in PCR reactions incorporating. OligodTAnchor and HDDADI.4. For 3' RACE human polyA+ RNA was primed with OligodTAnchor and reverse transcribed prior to PCR with oligos HDDAHI.5 and Anchor. All PCR products were cloned into pCRTOPO2.1 (In Vitrogen) following the manufactures instructions. CDNA inserts were sequenced using a T7 sequences kit (Amersham) according to the manufacturers instructions.

The sequence of human DDAHII was obtained by data base searching. The database of translated EMBL open reading frames (trembl) was searched with the rat DDAHI peptide sequence. This search identified a hypothetical mouse open reading frame (accession number 008972) that has the capacity to encode a protein of 228 amino acids with 63% similarity to rat DDAHI. Interogation of dbEST with the nucleotide sequence encoding the hypothetical mouse protein identified numerous overlapping human EST's which contained an open reading frame or 858bp with the potential to encode a 285 amino acid protein that is 52% identical to human DDAHI. The oligonucleotides used in these experiments are shown in Table 1.

**Table 1. Oligonucleotides used.**

| **Name** | **Sequence** | **Details** |
|---|---|---|
| HDDAHI.1 | GGT TGA CAT GAT GAA AGA AGC | Homologous to nucleotides 303-324 of human DDAHI |
| HDDAHI.2 | CAG CAC CCC GTT GAT TTG TC | Homologous to nucleotides 454-435 of human DDAHI |
| HDDAHI.3 | GCT TCT TIC ATC ATG TCA ACC | Homologous to nucleotides 324-303 of human DDAHI |
| HDDAHI.4 | CCC AAC AAA GGG CAC GTC TTG | Homologous to nucleotides 682-703 of human DDAHI |
| HDDAHII.1 | GAT CGA ATT CAG GAT GGG GAC GCC GGG G | Homologous to nucleotides -2-15 of human DDAHII encoding an upstream EcoR1 site |
| HDDAHII.2 | GAC TTC TAG AGC TGT GGG GGC GTG TG | Homologous to nucleotides 858-840 of human DDAHII encoding a downstream XbaI site |
| HDDAHII.3 | CTC AGC TCC CTC TGC TTG GTG | Homologous to nucleotides 813-834 of human DDAHII |
| HDDAHII.4 | GAG GGA GGA TTC ACC CAG TGG | Homologous to nucleotides 1003-1024 of human DDAHII |
| RDDAHI.1 | TCC GCG GGA TCC ATG GCC GGC CTC | Homologous to nucleotides -12-12 of rat DDAHI |
| RDDAHI.2 | CGC TCG GTC TAG ATC AAC AGT CTG TCT | T Homologous to nucleotides 872-844 of rat DDAHI |
| HNNOS.1 | CTG CTG ATG TCC TCA AAG CCA TCC | Homologous to nucleotides 4079-4102 of human nNOS |
| HNNOS.2 | TCT GTC CCG CGC TTA CAA ACT TGC | Homologous to nucleotides 4353-4330 of human nNOS |
| HENOS.1 | CAA CCA ACG TCC TGC AGA CCG TGC | Hemologous to nucleotides 3379-3402 of human eNOS |
| HENOS.2 | GGC GGA CCT GAG TCG GGC AGC CGC | Homologous to nucleotides 3690-3667 of human eNOS |
| Oligo d(T) Anchor | GAG CAC GCG TAT CGA TGT CGA CTT TTT TTT TTT TTT TTV | 5'/3' RACE oligo d(T) anchor primer |
| Anchor | GAC CAC GCG TAT CGA TGT CGA C | 5'/3' RACE anchor primer |

### Recombinant expression

The entire human DDAHII open reading frame was PCR amplified from oligo dT primed human kidney cDNA using oligos HDDAHII.1 and HDDAHII.2. Oligo HDDAH II.1 is complementary to base pairs 2-15 of the human DDAHII cDNA and contains an *Eco*RI site in frame with the *Eco*TI site of pPROX.HTa (Life Technologies). HDDAII.2 is complementary to base pairs 858-840 of the human DDAHII cDNA and contains an artificial *Xba*I site. PCR produced a single product of ∼850bp which was digested with *Eco*RI and *Xba*i, ligated into *Eco*RI and *Xba*I digested pRPROX.HTa and transformed into competent *E.coli* DH5α. A positive clone (pPDDAHII) containing an insert of 858bp was identified and the insert sequenced on both strands. For expression of recombinant human DDAHII, *E.coli* were grown in liquid culture at 25°C to an OD₆₀₀ of 0.5-0.6. Expression was then induced by the addition of IPTG to a final concentration of 1mM and incubation continued for a further two hours. Following induction, cells were collected by centrifugation, weighed and resuspended in ice cold assay buffer (100mM Na₃HPO₄ pH 6.5) at 1g cells/ml. Cells were disrupted by sonication (6 X 10secs, with 10 sec. intervals) and centriguged at 50,000g to separate soluble material from insoluble cell debris.

### DDAH Assay

Aliquots of *E.coli* lysates were incubated at 37 °C for 60 min. with 250 ml of 100mM Na₂HPO₄ pH 6.5 containing 0.02µCi [¹⁴C]L-NMMA as described previously (MacAllister et al., 1996, Br. J. Pharmacol. 115, 1001-1004). Following incubation samples were prepared for determination of [¹⁴C] citrulline production by scintillation counting. Reactions were vortexed with 1ml of 50% (w/v) dowex 50X8-400, centrifuged at 10,000g for 5 min and then 500µl of the supernatant was mixed with 5ml of liquid scintillation fluid and the [¹⁴C] content determined.

### DDAH Assay

Samples were assayed by incubating 100µl of cells lysates with an equal volume of assay mix (100mM Na₂HPO₄ pH 6.5, containing 0.02µCi [¹⁴C] L-NMMA and 100µM cold L-NMMA) at 37°C for 60 min, as previously reported. The samples were then prepared for scintillation counting to measure the production of [¹⁴C] citrulline by adding 400µl of 50% (w/v) Dowex 50X-400 to the reactions, vortexing and centrifugation at 13000g for 2 min. The [¹⁴C] content of 100µl of supernatant in 1ml scintillation fluid was then determined.

### Northern blot analysis

The tissue distribution of human DDAHI, DDAHII, endothelial NOS and neuronal NOS mRNA was determined by hybridization of ³²P-labelled cDNA probes to commercially available northern blot (Clontech, human multiple tissue northern blot). Probes were produced by PCR amplification of oligo dT-primed human kidney polyA+ mRNA using oligonucleotide primer pairs HDDAHI 4 and 5, HDDAHII 3 and 4, HENOS i and 2 and HNNOS 1 and 2. Following PCR reaction products were resolved on 2% agarose gels, isolated from the gel and labelled using a random primed labelling kit (Boehringer) according to the manufacturers instructions. Labelled probes were purified on Nick columns (Pharmacia) and hybridized to filters according to the manufacturers instructions.

### Database searching for bacterial sequences

The amino acid sequence of human DDAHI was added to search the expressed sequence tag database (dbEST). Open reading frames which showed significant similarity to this sequence were identified in *S.coelicolor* (46.5% over 163 amino acids), *P.auruginosa* (44.3% over 226 amino acids) and *M.tuberculosis* (37.5% over 24 amino acids). The cDNA sequences encoding these putative bacterial DDAHs were then used to design the primers ScDDAHI and ScDDAH2, PaDDAHs were then used to design the primers ScDDAHi and ScDDAH2, PaDDAH1 and PaDDAH4, and TbDDAH1 and TbDDAH4. These oligonucleotides PaDEIM2 and PaDEIM3 were designed from the cDNA sequence of *P.aeruginosa* arginine deiminase to amplifying the coding region. Primers TbDDAH1 and TbDDAH4 were designed to amplify an open reading frame from cosmid Y3G12 which was identified through the database search using hDDAH1. The oligonucleotides used in these experiments are shown in Table 2.

**Table 2. Oligonucleotides Used.**

| **Name** | **Sequence** | **Details** |
|---|---|---|
| ScDDAH 1 | GATCGAATTGTGCCCAGCAAGAAG GCCTG | Homologous to -9 to 20 encoding an upstream *Eco*RI site |
| ScDDAH 2 | GATCTCTAGATCAGTCGTACAGCTC GCGC | Homologous to 732 to 751 encoding a downstream *Xba*I site |
| PaDDAH 1 | GAATTCATGTTCAAGCACATCATCG | Homologous to 1 to 19 encoding an upstream *Eco*RI site |
| PaDDAH 4 | AAGCTTCCCCGCGGCATGGTTC | Homologous to 782 to 768 encoding a downstream *Hin*d III site |
| TbDDAH 1 | GAATTCCGCAATGTATCAATG G | Homologous to -12 to 4 encoding an upstream *Eco* RI site |
| TbDDAH 2 | AAGTTCCCACGCACCCTCAG | Homologous to 1024 to 1011 encoding a downstream *Hin*d III site |
| PaDEIM 2 | GAATTCAGCACGGAAAAACCAAAC | Homologous to 3 to 22 encoding an upstream *Eco* RI site |
| PaDEIM 3 | AAGCTTGTAGTCGATCGGGTCGC | Homologous to 1257 to 1239 encoding a downstream *Hind* III site |

### Polymerase Chain Reaction and cDNA Cloning

Amplification of *S.coelicolar* DDAH from cosmid 4C6 was carried out by PCR using the oligonucleotides ScDDAH1 and ScDDAH2. PCR was carried out on *P.aeruginosa* genomic DNA using the primers PADDAG1 and PADDAG4 to amplify the putative DDAH. The *P.aeruginosa* arginine deiminase was also amplified using the oligonucleotides PaDEIM2 and PaDEIM3. The oligonucleotides TbDDAH1 and TbDDAH4 were used in PCR to amplifying the *M.tuberculosis* DDAH from cosmid Y3G12 DNA.

All PCR products were cloned into pCRTOPO2.1 (InVitrogen) following the manufacturer's instructions.

### Expression of Recombinant Proteins

The inserts containing the open reading frames of the bacterial DDAHs were excised from the vector using *Eco* RI and *Hind* III, gel purified, ligated into *Eco* RI and *Hind* III digested pProEX.HT and transformed into competent *E. coli* DH5α. The arginine deiminase was treated as above but was cloned into *Eco*RI and *Hind* III digested pBAD B (InVitrogen).

For expression of the recombinant proteins, a positive clone was picked and grown in liquid media supplemented with 100 µg/ml ampicillin. *E. coli* were grown at 25°C to an OD₆₀₀ of 0.5-0.6 for the bacterial DDAHs in pProEX.HT, and at 37° for the arginine deiminase in pBAD B. Induction of expression of the bacterial DDAHs was carried out by addition of IPTG to a final concentration of 1mM and a further incubation of 2 hours at 25°C. Expression of the arginine deiminase was induced by adding arabinose to a final concentration of 0.02% (w/v) and incubating fora further 4 hours at 37°C.

After induction, cells were harvested by centrifugation and resuspended to a concentration of 250 mg/ml in assay buffer (100mM Na₂HPO₄, pH6.5). Cells were disrupted by sonication (6 X 10 secs.) And centrifuged at 18,000g to remove particulate material.

### SDS-Polyacrylamide Gel Electrophoresis and Western Blot Analysis

SDS-PAGE was performed in Tris/glycine buffer, pH8.3, on 12% (w/v) separating gel with a 3.5% (w/v) stacking gel. Proteins were transferred onto an Immobilon-P membrane (Millipore) at 200A for 30 minutes. Membranes were then blocked in 5% (w/v) milk in phosphate buffered saline with 0.1 Tween 20 (PBST) for 2 hours. The blot was probed with a polyHistidine antibody (Sigma) at a dilution of 1:3000 followed by anti-mouse Ig antibody coupled to horseradish peroxide (Amersham) at a dilution of 1:5000 then developed using an ECL chemiluminescence kit (Amersham).

### Results

### Cloning of human DDAHI and DDAHII

Using a combination of RT-PCR and RACE, a cDNA encoding the entire open reading frame of human DDAHI was assembled. The 858bp open reading frame is 90% homologous to rat DDAHI ORF (data not shown) and encodes a polypeptide of 285 amino acids that is 95% identical to the rat protein (Figure 1). A search of the 'trembl' data base using the rat DDAHI amino acid sequence identified a mouse open reading frame encoding a protein with 63% homology over 228 amino acids to rat DDAH. Further data base searching identified a human cDNA of 2000bp containing an open reading frame of 858bp with the potential to encode a protein of 285 amino acids (subsequently referred to as DDAHII). This open reading frame was 63% homologous to human DDAHI at the nucleotide level (data not shown) and the predicted protein is 62% similar to human DDAHI at the amino acid level (Figure 1). Like DDAHI, DDAHII appears to be highly conserved across mammalian species with 98& homology between murine and human DDAHII amino acid sequences (data not shown).

### Recombinant expression of human DDAHII

An N-terminally 6X His-tagged body of DDAHII was expressed in *E.coli* under the control of an IPTG induceable promoter. Following induction, a band of ∼40kDa (-35kDa human DDAHII + 4kDa 6X His-tag and linker) was apparent in the soluble fraction of cell lysates (Figure 2). The induced protein of ∼40kDa is specifically recognised by an anti-His6 antibody confirming its identity as recombinant human DDAHII (Figure 2). In order to establish whether DDAHII is a functional homologue of DDAHI we assayed bacterial cell lysates for DDAH activity. Lysates of cells transfected with empty vector did not metabolise [¹⁴C] L-NMMA. In contrast, lysates of cells expression recombinant DDAHII did metabolise [¹⁴C] L-NMMA (Figure 3). This action was inhibited by the DDAH inhibitor S-2-amino-4(3-methylguanidino) butanoic acid (4124W) [ref] and by competition with a molar excess of cold L-NMMA, ADMA or citrulline. Enzyme activity was unaffected in the presence of a molar excess of cold SDMA.

### Tissue distribution of human DDAH and NOS

To determine the tissue distribution of DDAHI and DDAHII messenger RNA and to explore any correlation between DDAH and NOS isoform expression we probed a commercially available human multiple tissue northern blots with labelled cDNA probed specific for each isoform (Figure 4). A DDAHI cDNA probe hybridized to a single band of ∼4.4Kb that was highly expressed in kidney, brain, pancreas and liver. Lower level expression was also clearly apparent in skeletal muscle whilst signals from the heart placenta and lung were barely detectably. In contrast, a cDNA probe for DDAHII hybridized to a single band of ∼2Kb that was most highly expressed by heart, kidney and placenta. In the case of DDAHII, lower level expression in the brain was barely detectable. A probe specific for nNOS revealed high level expression in skeletal muscle and brain, lower levels in kidney and pancreas with no detectable expression in heart, placenta, lung and liver. Endothelial NOS was highly expressed in placenta and heart with lower levels apparent in skeletal muscle, liver, kidney, pancreas and lung, whilst expression in brain was undetectable. The level of β-actin message in each lane is shown as an indication of mRNA loading.

To extend this analysis of DDAH isoform distribution we have examined the level of DDAHI and II mRNA in 43 adult and 7 fetal tissues by RNA dot blot analysis using isoform specific probes. This analysis indicated that in the brain DDAHI is expressed at high levels whilst DDAHII is expressed at relatively low levels, with both isoforms being expressed at high levels in the spinal cord. However, in highly vascularised tissues such as the heart, aorta, placenta and lung DDAHII expression clearly predominates over DDAHI. In immune tissues (spleen, thymus, peripheral leukocyte, lymph node and bone marrow) DDAHII expression is clearly apparent whilst DDAHI expression is barely detectable. This distinct pattern of DDAH expression is consistent with the suggestion above that DDAHI predominates in tissues which express high levels of the neuronal isoform of NOS and DDAHII expression is most marked in tissues that also express the endothelial form of NOS.

### The chromosomal location of human DDAHI and II

In order to determine the chromosomal location of the human DDAHI and DDAHII genes we employed radiation hybrid mapping and fluorescent *in situ* hybridisation (FISH) techniques. For radiation hybrid mapping we screened the Genebridge 4 Radiation Hybrid DNA Panel (Originators Peter Goodfellow and Jean Weissenbach, obtained from the UK HGMP Resource Centre) using oligonucleotide pairs to amplify nucleotides 47 to 273 of the DDAHI cDNA and nucleotides -16 to -163 of the DDAHII cDNA. The results were submitted to the HGMP RhyME mapper which mapped DDAHI to chromosome 1 (LOD=2.7) and DDAHII to chromosome 6p21-23 (LOD>3). For FISH mapping, a human DDAHI genomic clone (clone no. 2218H15, accession number AQ145822) was identified by a blast search of the GSS database using the human DDAHI cDNA sequence. A genomic clone (clone no. 84h3)cantaining the human DDAHII gene was isolated by PCR screening of a human genomic PAC library (RPCI1, onstructed by Pieter de Jong and obtained from the UK HGMP resource Centre). Clones 2218H15 and 84h3 were used as probes in FISH which localised DDAHI to chromosome 1p22 and DDAHII to chromosome 6p21.3.

### Identification of DDAH-related proteins

In order to identity proteins with significant primary sequence homology to DDAHI/II we performed a search of the swissprot data with both the human DDAHI and DDAHII protein sequences. This search revealed significant homology between both DDAH sequences and the sequences of arginine deiminase enzymes from several microbial species. The highest degree of homology was found with the sequence of arginine deiminase from *Pseudomonas putida* (Accession no. p41142) (Figure 5). The homology was strongest within a 69 amino acid domain (residues 123 to 191 of DDAHI) where the identify rises to 22% and the similarity to 70%. In this domain, DDAHI and DDAHII are 80% identical. Comparison of the sequences of human DDAHI and DDAHI with other arginine-utilizing or arginine-producing enzymes, such as peptidyl-arginine deiminase, arginase, argininosuccinate lysase, arginine decarboxylase and nitric oxide synthase revealed no significant amino acid homology.

### Cloning of Streptomyces and Pseudomonas DDAH

A ClustalW alignment of the DDAHs from *S. coelicolor, P. aeruginosa, M. tuberculosis* and human DDAH I amino acid sequences is shown in Figure 6A. Alignments of *P. aeruginosa* DDAH and arginine deiminase are also shown in Fig. 6B.

Oligonucleotides ScDDAH 1 and ScDDAH 2 were designed from the open reading frame of a putative *S. coelicolor* DDAH identified through database Screening. These primers gave a PCR product of approximately 850bp. The primers PaDDAH 1 and PaDDAH 4 amplified a product of approximately 780bp from *P. aeruginosa* genomic DNA and TbDDAH 1 and TbDDAH 4 gave a PCR product of approximately 1150bp from the cosmid Y3G12.

### Expression of Recombinant Bacterial DDAHs

Expression of N-terminally 6X His-tagged forms of *S. coelicolor, M. tuberculosis* and *P. aeruginosa* DDAH was carried out in *E. coli* under the control of an IPTG inducible promoter. Following induction, a band of ∼36kDa was observed in *S. coelicolor* (∼32kDa *S. coelicolor* DDAH + ∼4kDa 6X His-tag) cell lysates and of 33kDa in *P. aeruginosa* (29kDa *P. aeruginosa* DDAH + ∼4kDa 6X His-tag) cell lysates. A polyHistidine antibody specifically recognized these bands providing confirmation of the identity of these proteins as recombinant *S. coelicolor* and *P. aeruginosa* DDAH respectively.

### Activity of Recombinant Bacterial DDAH proteins

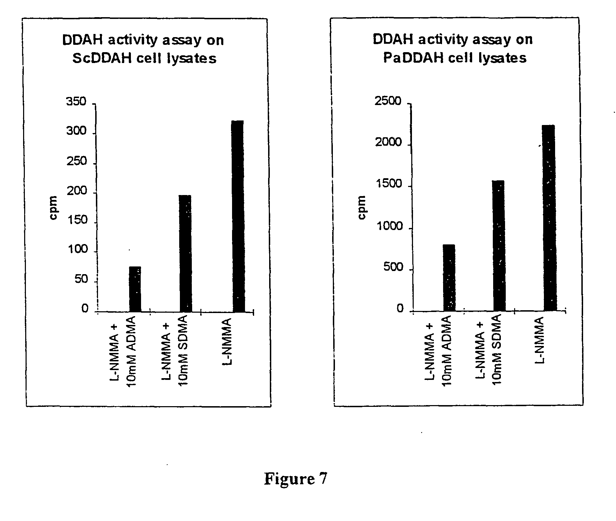

The bacterial DDAH cell lysates were assayed for DDAH activity to determine whether they were functional homologues of human DDAH I. These were found to metabolize [14C] L-NMMA, as shown in Figure 7. Empty vector was also transfected into cells and the lysates from these were found not to metabolize [14C] L-NMMA. *P. aeruginosa* DDAH showed higher activity compared to that of *S. coelicolor* DDAH.

ADMA and SDMA were both found to compete with L-NMMA as substrates for the bacterial DDAHs (Fig. 7) with ADMA showing a greater effect than SDMA on the metabolism of L-NMMA. Similar results have been obtained for DDAH from *M.tuberculosis.*

## Claims

1. A polynucleotide which:
(a) encodes a polypeptide that has methylarginase activity, which polynucleotide is selected from:
(1) the coding sequence of SEQ ID NO: 3; and
(2) a sequence that is degenerate as a result of the genetic code with respect to a sequence defined in (1); or
(b) is a sequence complementary to a polynucleotide defined in (a).

2. A polynucleotide according to claim 1 which is a DNA sequence.

3. A polynucleotide according to claim 1 or 2 which encodes the amino acid sequence of SEQ ID NO: 4.

4. A polypeptide which has methylarginase activity and which comprises the sequence set out in SEQ ID NO: 4.

5. A vector incorporating a polynucleotide according to any one of claims 1 to 3.

6. A vector according to claim 5, which is an expression vector.

7. A cell harbouring a vector according to claim 5 or 6.

8. A process for the preparation of a polypeptide which has methylarginase activity, which process comprises cultivating a host cell harbouring an expression vector according to claim 6 under conditions to provide for expression of the said polypeptide, and recovering the expressed polypeptide.

9. An antibody capable of binding a polypeptide which has methylarginase activity and which comprises the sequence set out in SEQ ID NO: 4, wherein the antibody is able to discriminate between a DDAHI polypeptide and a DDAHII polypeptide.

10. A transgenic non-human animal which is not capable of expressing an active form of a dimethylarginine dimethylaminohydrolase II (DDAHII), wherein, in the transgenic animal, polynucleotide sequence from the DDAHII gene locus has been deleted or replaced with polynucleotide sequence from another locus or another organism or in which the coding sequence of the DDAHII gene has been altered such that the expressed polypeptide shows no methylarginase activity; and wherein said non-human animal is a mammal

11. A non-human animal according to claim 10 which is a mouse.

12. A method for identifying a modulator of methylarginase activity and/or expression, which method comprises:
(i) contacting a polynucleotide according to any one of claims 1 to 3, a polypeptide according to claim 4, a vector according to claim 6 or a cell according to claim 7 and a test substance under conditions that would permit methylarginase activity in the absence of the test substance; and
(ii) determining thereby whether the said substance modulates the activity and/or expression of methylarginase.

13. A polynucleotide according to any of claims 1 to 3, a polypeptide according to claim 4 or an expression vector according to claim 6 for use in a method of treatment of the human or animal body by therapy.

14. A polynucleotide according to any one of claims 1 to 3, a polypeptide according to claim 4 or an expression vector according to claim 6 for use in a method of treatment of hyperlipidaemia, renal failure, hypertension, restenosis after angioplasty, atherosclerosis, complications of heart failure, schizophrenia, multiple sclerosis or cancer.

15. Use of a polynucleotide according to any one of claims 1 to 3, a polypeptide according to claim 4 or an expression vector according to claim 6 for the manufacture of a medicament for use in the treatment of hyperlipidaemia, renal failure, hypertension, restenosis after angioplasty, atherosclerosis, complications of heart failure, schizophrenia, multiple sclerosis or cancer.

16. A pharmaceutical composition comprising a polynucleotide according to any one of claims 1 to 3, a polypeptide according to claim 4 or an expression vector according to claim 6 and a pharmaceutically acceptable carrier and/or diluent.

## Patentansprüche

1. Polynucleotid, das:
(a) für ein Polypeptid kodiert, das Methylarginase-Aktivität aufweist,
wobei das Polynucleotid ausgewählt ist aus:
(1) der Kodierungssequenz von SEQ ID NO: 3; und
(2) einer Sequenz, die ein Degenerationsprodukt als Ergebnis des genetischen Codes in Bezug auf die in (1) definierte Sequenz ist; oder
(b) eine Sequenz ist, die zu dem unter (a) definierten Polynucleotid komplementär ist.

2. Polynucleotid nach Anspruch 1, bei dem es sich um eine DNA-Sequenz handelt.

3. Polynucleotid nach Anspruch 1 oder 2, das für die Aminosäuresequenz von SEQ ID NO: 4 kodiert.

4. Polypeptid, das Methylarginase-Aktivität aufweist und die in SEQ ID NO: 4 aufgeführte Sequenz umfasst.

5. Vektor, der ein Polynucleotid nach einem der Ansprüche 1 bis 3 beinhaltet.

6. Vektor nach Anspruch 5, bei dem es sich um einen Expressionsvektor handelt.

7. Zelle, in der ein Vektor nach Anspruch 5 oder 6 enthalten ist.

8. Verfahren zur Herstellung eines Polypeptids, das Methylarginase-Aktivität aufweist, wobei das Verfahren die Züchtung einer Wirtszelle, in der ein Expressionsvektor nach Anspruch 6 enthalten ist, unter Bedingungen, die für eine Expression des Polypeptids sorgen, und das Gewinnen des exprimierten Polypeptids umfasst.

9. Antikörper, der zur Bindung eines Polypeptids, das Methylarginase-Aktivität aufweist und die in SEQ ID NO: 4 aufgeführte Sequenz umfasst, befähigt ist, wobei der Antikörper zwischen einem DDAHI-Polypeptid und einem DDAHII-Polypeptid unterscheiden kann.

10. Transgenes, nicht-humanes Tier, das nicht zur Expression einer aktiven Form einer Dimethylarginin-dimethylaminohydrolase II (DDAHII) befähigt ist, wobei im transgenen Tier die Polynucleotidsequenz aus dem DDAHII-Genlocus deletiert oder mit einer Polynucleotidsequenz aus einem anderen Locus oder einem anderen Organismus ersetzt worden ist oder bei dem die Kodierungssequenz des DDAHII-Gens so verändert worden ist, dass das exprimierte Polypeptid keine Methylarginase-Aktivität aufweist und wobei es sich beim nicht-humanen Tier um einen Säuger handelt.

11. Nicht-humanes Tier nach Anspruch 10, bei dem es sich um eine Maus handelt.

12. Verfahren zum Identifizieren eines Modulators von Methylarginase-Aktivität und/oder von deren Expression, wobei das Verfahren folgendes umfasst:
(i) das Kontaktieren eines Polynucleotids nach einem der Ansprüche 1 bis 3, eines Polypeptids nach Anspruch 4, eines Vektors nach Anspruch 6 oder einer Zelle nach Anspruch 7 und einer Testsubstanz unter Bedingungen, die in Abwesenheit der Testsubstanz Methylarginase-Aktivität ermöglichen würden; und
(ii) das Feststellen, ob die Testsubstanz die Aktivität und/oder Expression von Methylarginase moduliert.

13. Polynucleotid nach einem der Ansprüche 1 bis 3, Polypeptid nach Anspruch 4 oder Expressionsvektor nach Anspruch 6 zur Verwendung in einem Verfahren zur therarpeutischen Behandlung des menschlichen oder tierischen Körpers.

14. Polynucleotid nach einem der Ansprüche 1 bis 3, Polypeptid nach Anspruch 4 oder Expressionsvektor nach Anspruch 6 zur Verwendung in einem Verfahren zur Behandlung von Hyperlipidämie, Nierenversagen, Hochdruck, Restenose nach Angioplastie, Atherosklerose, Komplikationen bei Herzinsuffizienz, Schizophrenie, multipler Sklerose oder Krebs.

15. Verwendung eines Polynucleotids nach einem der Ansprüche 1 bis 3, eines Polypeptids nach Anspruch 4 oder eines Expressionsvektors nach Anspruch 6 zur Herstellung eines Arzneimittels zur Verwendung bei der Behandlung von Hyperlipidämie, Nierenversagen, Hochdruck, Restenose nach Angioplastie, Atherosklerose, Komplikationen bei Herzinsuffizienz, Schizophrenie, multipler Sklerose oder Krebs.

16. Pharmazeutische Zusammensetzung, umfassend ein Polynucleotid nach einem der Ansprüche 1 bis 3, ein Polypeptid nach Anspruch 4 oder einen Expressionsvektor nach Anspruch 6 und einen pharmazeutisch verträglichen Träger und/oder Verdünnungsmittel.

## Revendications

1. Polynucléotide qui :
(a) code un polypeptide qui a une activité méthylarginase, lequel polynucléotide est sélectionné parmi :
(1) la séquence codante de SEQ ID NO:3 ; et
(2) une séquence dégénérée par suite du code génétique par rapport à une séquence définie en (1) ; ou
(b) est une séquence complémentaire d'un polynucléotide défini en (a).

2. Polynucléotide selon la revendication 1 qui est une séquence d'ADN.

3. Polynucléotide selon la revendication 1 ou 2 qui code la séquence d'acides aminés de SEQ ID NO:4.

4. Polypeptide qui a une activité méthylarginase et qui comprend la séquence présentée dans SEQ ID NO:4.

5. Vecteur incorporant un polynucléotide selon l'une quelconque des revendications 1 à 3.

6. Vecteur selon la revendication 5, qui est un vecteur d'expression.

7. Cellule abritant un vecteur selon la revendication 5 ou 6.

8. Procédé de préparation d'un polypeptide qui a une activité méthylarginase, lequel procédé comprend la culture d'une cellule hôte abritant un vecteur d'expression selon la revendication 6 dans des conditions permettant l'expression dudit polypeptide, et la récupération du polypeptide exprimé.

9. Anticorps capable de se lier à un polypeptide qui a une activité méthylarginase et qui comprend la séquence présentée dans SEQ ID NO:4, où l'anticorps est capable de faire la distinction entre un polypeptide DDAHI et un polypeptide DDAHII.

10. Animal transgénique non humain qui n'est pas capable d'exprimer une forme active d'une diméthylarginine diméthylaminohydrolase II (DDAHII), où, chez l'animal transgénique, la séquence polynucléotidique provenant du locus génique de DDAHII a été supprimée ou remplacée par une séquence polynucléotidique d'un autre locus ou d'un autre organisme ou dans lequel la séquence codante du gène de DDAHII a été modifiée de façon telle que le polypeptide exprimé ne présente pas d'activité méthylarginase, et où ledit animal non humain est un mammifère.

11. Animal non humain selon la revendication 10, qui est une souris.

12. Procédé d'identification d'un modulateur de l'activité et/ou de l'expression de la méthylarginase, lequel procédé comprend les étapes consistant à :
(i) mettre en contact un polynucléotide selon l'une quelconque des revendications 1 à 3, un polypeptide selon la revendication 4, un vecteur selon la revendication 6 ou une cellule selon la revendication 7 et une substance à tester dans des conditions qui permettent l'activité méthylarginase en l'absence de la substance à tester ; et
(ii) déterminer si ladite substance module l'activité et/ou l'expression de la méthylarginase.

13. Polynucléotide selon l'une quelconque des revendications 1 à 3, polypeptide selon la revendication 4 ou vecteur d'expression selon la revendication 6 pour utilisation dans un procédé de traitement de l'organisme humain ou animal par une thérapeutique.

14. Polynucléotide selon l'une quelconque des revendications 1 à 3, polypeptide selon la revendication 4 ou vecteur d'expression selon la revendication 6 pour utilisation dans un procédé de traitement de l'hyperlipidémie, de l'insuffisance rénale, de l'hypertension, de la resténose après angioplastie, de l'athérosclérose, des complications de l'insuffisance cardiaque, de la schizophrénie, de la sclérose en plaques ou du cancer.

15. Utilisation d'un polynucléotide selon l'une quelconque des revendications 1 à 3, d'un polypeptide selon la revendication 4 ou d'un vecteur d'expression selon la revendication 6 pour la préparation d'un médicament pour utilisation dans le traitement de l'hyperlipidémie, de l'insuffisance rénale, de l'hypertension, de la resténose après angioplastie, de l'athérosclérose, des complications de l'insuffisance cardiaque, de la schizophrénie, de la sclérose en plaques ou du cancer.

16. Composition pharmaceutique comprenant un polynucléotide selon l'une quelconque des revendications 1 à 3, un polypeptide selon la revendication 4 ou un vecteur d'expression selon la revendication 6 et un véhicule et/ou diluant pharmaceutiquement acceptables.


### SEQUENCE LISTING

(1) GENERAL INFORMATION:
   (i) APPLICANT:
      (A) NAME: UNIVERSITY COLLEGE LONDON
      (B) STREET: Gower Street
      (C) CITY: London
      (E) COUNTRY: United Kingdom
      (F) POSTAL CODE (ZIP): WC1E 6BT
   (ii) TITLE OF INVENTION: SCREEN METHOD
   (iii) NUMBER OF SEQUENCES: 12
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0. Version #1.30 (EPO)
(2) INFORMATION FOR SEQ ID NO: 1:
   (i) SEQUENCE CHARACTERISTICS:
      (A) LENGTH: 858 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 1:
   ATGGCCGGCC TCGGCCACCC CTCCGCCTTC GGCCGGGCCA CCCACGCCGT GGTGCGGGCG    60
   CTACCCGAGT CGCTCTGCCA GCACGCGCTG AGAAGCGCCA AGGGCGAGGA GGTGGACGTC   120
   GCCCGCGCGG AACGGCAGCA CCAGCTCTAC GTGGGCGTGC TGGGCAGCAA GCTGGGGCTG   180
   CAGGTGGTGG AGCTGCCGGC CGACGAGAGC CTTCCGGACT GCGTCTTCGT GGAGGACGTG   240
   GCCGTGGTGT GCGAGGAGAC GGCCCTCATC ACCCGACCCG GGGCGCCGAG CCGGAGGAAG   300
   GAGGTTGACA TGATGAAAGA AGCATTAGAA AAACTTCAGC TCAATATAGT AGAGATGAAA   360
   GATGAAAATG CAACTTTAGA TGGCGGAGAT GTTTTATTCA CAGGCAGAGA ATTTTTTGTG   420
   GGCCTTTCCA AAAGGACAAA TCAACGAGGT GCTGAAATCT TGGCTGATAC TTTTAAGGAC   480
   TATGCAGTCT CCACAGTGCC AGTGGCAGAT GGGTTGCATT TGAAGAGTTT CTGCAGCATG   540
   GCTGGGCCTA ACCTGATCGC AATTGGGTCT AGTGAATCTG CACAGAAGGC CCTTAAGATC   600
   ATGCAACAGA TGAGTGACCA CCGCTACGAC AAACTCACTG TGCCTGATGA CATAGCAGCA   660
   AACTGTATAT ATCTAAATAT CCCCAACAAA GGGCACGTCT TGCTGCACCG AACCCCGGAA   720
   GAGTATCCAG AAAGTGCAAA GGTTTATGAG AAACTGAAGG ACCATATGCT GATCCCCGTG   780
   AGCATGTCTG AACTGGAAAA GGTGGATGGG CTGCTCACCT GCTGCTCAGT TTTAATTAAC   840
   AAGAAGGTAG ACTCCTGA                                                 858
(2) INFORMATION FOR SEQ ID NO: 2:
   (i) SEQUENCE CHARACTERISTICS:
      (A) LENGTH: 285 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 2:
   Met Ala Gly Leu Gly His Pro Ser Ala Phe Gly Arg Ala Thr His Ala
   1               5                   10                  15
   Val Val Arg Ala Leu Pro Glu Ser Leu Cys Gln His Ala Leu Arg Ser
               20                  25                  30
   Ala Lys Gly Glu Glu Val Asp Val Ala Arg Ala Glu Arg Gln His Gln
           35                  40                  45
   Leu Tyr Val Gly Val Leu Gly Ser Lys Leu Gly Leu Gln Val Val Glu
       50                  55                  60
   Leu Pro Ala Asp Glu Ser Leu Pro Asp Cys Val Phe Val Glu Asp Val
   65                  70                  75                  80
   Ala Val Val Cys Glu Glu Thr Ala Leu Ile Thr Arg Pro Gly Ala Pro
                   85                  90                  95
   Ser Arg Arg Lys Glu Val Asp Met Met Lys Glu Ala Leu Glu Lys Leu
               100                 105                 110
   Gln Leu Asn Ile Val Glu Met Lys Asp Glu Asn Ala Thr Leu Asp Gly
           115                 120                 125
   Gly Asp Val Leu Phe Thr Gly Arg Glu Phe Phe Val Gly Leu Ser Lys
       130                 135                 140
   Arg Thr Asn Gln Arg Gly Ala Glu Ile Leu Ala Asp Thr Phe Lys Asp
   145                 150                 155                 160
   Tyr Ala Val Ser Thr Val Pro Val Ala Asp Gly Leu His Leu Lys Ser
                   165                 170                 175
   Phe Cys Ser Met Ala Gly Pro Asn Leu Ile Ala Ile Gly Ser Ser Glu
               180                 185                 190
   Ser Ala Gln Lys Ala Leu Lys Ile Met Gln Gln Met Ser Asp His Arg
           195                 200                 205
   Tyr Asp Lys Leu Thr Val Pro Asp Asp Ile Ala Ala Asn Cys Ile Tyr
       210                 215                 220
   Leu Asn Ile Pro Asn Lys Gly His Val Leu Leu His Arg Thr Pro Glu
   225                 230                 235                 240
   Glu Tyr Pro Glu Ser Ala Lys Val Tyr Glu Lys Leu Lys Asp His Met
                   245                 250                 255
   Leu Ile Pro Val Ser Met Ser Glu Leu Glu Lys Val Asp Gly Leu Leu
               260                 265                 270
   Thr Cys Cys Ser Val Leu Ile Asn Lys Lys Val Asp Ser
           275                 280                 285
(2) INFORMATION FOR SEQ ID NO: 3:
   (i) SEQUENCE CHARACTERISTICS:
      (A) LENGTH: 858 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 3:
   ATGGGGACGC CGGGGGAGGG GCTGGGCCGC TGCTCCCATG CCCTGATCCG GGGAGTCCCA    60
   GAGAGCCTGG CGTCGGGGGA AGGTGCGGGG GCTGGCCTTC CCGCTCTGGA TCTGGCCAAA   120
   GCTCAAAGGG AGCACGGGGT GCTGGGAGGT AAACTGAGGC AACGACTGGG GCTACAGCTG   180
   CTAGAACTGC CACCTGAGGA GTCATTGCCG CTGGGACCGC TGCTTGGCGA CACGGCCGTG   240
   ATCCAAGGGG ACACGGCCCT AATCACGCGG CCCTGGAGCC CCGCTCGTAG GCCAGAGGTC   300
   GATGGAGTCC GCAAAGCCCT GCAAGACCTG GGGCTCCGAA TTGTGGAAAT AGGAGACGAG    360
   AACGCGACGC TGGATGGCAC TGACGTTCTC TTCACCGGCC GGGAGTTTTT CGTAGGCCTC    420
   TCCAAATGGA CCAATCACCG AGGAGCTGAG ATCGTGGCGG ACACGTTCCG GGACTTCGCC    480
   GTCTCCACTG TGCCAGTCTC GGGTCCCTCC CACCTGCGCG GTCTCTGCGG CATGGGGGGA    540
   CCTCGCACTG TTGTGGCAGG CAGCAGCGAC GCTGCCCAAA AGGCTGTCCG GGCAATGGCA    600
   GTGCTGACAG ATCACCCATA TGCCTCCCTG ACCCTCCCAG ATGACGCAGC TGCTGACTGT    660
   CTCTTTCTTC GTCCTGGGTT GCCTGGTGTG CCCCCTTTCC TCCTGCACCG TGGAGGTGGG    720
   GATCTGCCCA ACAGCCAGGA GGCACTGCAG AAGCTCTCTG ATGTCACCCT GGTACCTGTG    780
   TCCTGCTCAG AACTGGAGAA AGCTGGCGCC GGGCTCAGCT CCCTCTGCTT GGTGCTCAGC    840
   ACACGCCCCC ACAGCTGA                                                  858
(2) INFORMATION FOR SEQ ID NO: 4:
   (i) SEQUENCE CHARACTERISTICS:
      (A) LENGTH: 285 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 4:
   Met Gly Thr Pro Gly Glu Gly Leu Gly Arg Cys Ser His Ala Leu Ile
   1               5                   10                  15
   Arg Gly Val Pro Glu Ser Leu Ala Ser Gly Glu Gly Ala Gly Ala Gly
               20                  25                  30
   Leu Pro Ala Leu Asp Leu Ala Lys Ala Gln Arg Glu His Gly Val Leu
           35                  40                  45
   Gly Gly Lys Leu Arg Gln Arg Leu Gly Leu Gln Leu Leu Glu Leu Pro
       50                  55                  60
   Pro Glu Glu Ser Leu Pro Leu Gly Pro Leu Leu Gly Asp Thr Ala Val
   65                  70                  75                  80
   Ile Gln Gly Asp Thr Ala Leu Ile Thr Arg Pro Trp Ser Pro Ala Arg
                   85                  90                  95
   Arg Pro Glu Val Asp Gly Val Arg Lys Ala Leu Gln Asp Leu Gly Leu
               100                 105                 110
   Arg Ile Val Glu Ile Gly Asp Glu Asn Ala Thr Leu Asp Gly Thr Asp
           115                 120                 125
   Val Leu Phe Thr Gly Arg Glu Phe Phe Val Gly Leu Ser Lys Trp Thr
       130                 135                 140
   Asn His Arg Gly Ala Glu Ile Val Ala Asp Thr Phe Arg Asp Phe Ala
   145                 150                 155                 160
   Val Ser Thr Val Pro Val Ser Gly Pro Ser His Leu Arg Gly Leu Cys
                   165                 170                 175
   Gly Met Gly Gly Pro Arg Thr Val Val Ala Gly Ser Ser Asp Ala Ala
               180                 185                 190
   Gln Lys Ala Val Arg Ala Met Ala Val Leu Thr Asp His Pro Tyr Ala
           195                 200                 205
   Ser Leu Thr Leu Pro Asp Asp Ala Ala Ala Asp Cys Leu Phe Leu Arg
       210                 215                 220
   Pro Gly Leu Pro Gly Val Pro Pro Phe Leu Leu His Arg Gly Gly Gly
   225                 230                 235                 240
   Asp Leu Pro Asn Ser Gln Glu Ala Leu Gln Lys Leu Ser Asp Val Thr
                   245                 250                 255
   Leu Val Pro Val Ser Cys Ser Glu Leu Glu Lys Ala Gly Ala Gly Leu
               260                 265                 270
   Ser Ser Leu Cys Leu Val Leu Ser Thr Arg Pro His Ser
           275                 280                 285
(2) INFORMATION FOR SEQ ID NO: 5:
   (i) SEQUENCE CHARACTERISTICS:
      (A) LENGTH: 777 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 5:
   GTGCCCAGCA AGAAGGCCCT GGTCCGCCGC CCCAGCCCCA GGCTCGCCGA AGGACTGGTG     60
   ACACACGTCG AGCGGGAGCA GGTCGATCAC GGCCTGGCCC TCGAACAGTG GGACGCCTAC    120
   GTCGAGGCCC TCGGAGCACA CGGCTGGGAG ACTCTGGAGG TGGACCCGGC CGAGTACTGT    180
   CCGGACTCGG TCTTCGTCGA GGACGCCGTC GTCGTGTTCC GCAACGTCGC GCTGATCACG    240
   CGGCCCGGCG CCGAGTCGCG GCGCGCGGAG ACGGCCGGCG TCGAGGAGGC CGTGGCCCGG    300
   CTCGGCTGCT CGGTGAACTG GGTGTGGGAG CCGGGCACCC TGGACGGCGG CGACGTCCTG    360
   AAGATCGGCG ACACGATCTA CGTGGGACGC GGCGGCCGGA CCAACGCGGC CGGTGTCCAG    420
   CAGTTGCGGG CGGCGTTCGA GCCGCTGGGC GCCCGGGTCG TCGCCGTGCC CGTGAGCAAG    480
   GTGCTGCATC TGAAGTCGGC GGTCACCGCG CTGCCGGACG GGACGGTGAT CGGGCACATC    540
   CCGCTGACGG ACGTGCCCTC GCTGTTCCCC CGTTTCCTGC CGGTGCCGGA GGAGTCGGGG    600
   GCGCACGTGG TGCTGCTCGG CGGGAGCAGG CTGCTGATGG CGGCGAGCGC GCCCAAGACG    660
   GCGGAGCTGC TCGCCGATCT CGGTCACGAG CCGGTGCTCG TCGACATCGG GGAGTTCGAG    720
   AAGCTGGAGG GCTGTGTGAC GTGCCTCTCG GTCAGGCTGC GCGAGCTGTA CGACTGA       777
(2) INFORMATION FOR SEQ ID NO: 6:
   (i) SEQUENCE CHARACTERISTICS:
      (A) LENGTH: 258 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 6:
   Val Pro Ser Lys Lys Ala Leu Val Arg Arg Pro Ser Pro Arg Leu Ala
   1               5                   10                  15
   Glu Gly Leu Val Thr His Val Glu Arg Glu Gln Val Asp His Gly Leu
               20                  25                  30
   Ala Leu Glu Gln Trp Asp Ala Tyr Val Glu Ala Leu Gly Ala His Gly
           35                  40                  45
   Trp Glu Thr Leu Glu Val Asp Pro Ala Glu Tyr Cys Pro Asp Ser Val
       50                  55                  60
   Phe Val Glu Asp Ala Val Val Val Phe Arg Asn Val Ala Leu Ile Thr
   65                  70                  75                  80
   Arg Pro Gly Ala Glu Ser Arg Arg Ala Glu Thr Ala Gly Val Glu Glu
                   85                  90                  95
   Ala Val Ala Arg Leu Gly Cys Ser Val Asn Trp Val Trp Glu Pro Gly
               100                 105                 110
   Thr Leu Asp Gly Gly Asp Val Leu Lys Ile Gly Asp Thr Ile Tyr Val
           115                 120                 125
   Gly Arg Gly Gly Arg Thr Asn Ala Ala Gly Val Gln Gln Leu Arg Ala
       130                 135                 140
   Ala Phe Glu Pro Leu Gly Ala Arg Val Val Ala Val Pro Val Ser Lys
   145                 150                 155                 160
   Val Leu His Leu Lys Ser Ala Val Thr Ala Leu Pro Asp Gly Thr Val
                   165                 170                 175
   Ile Gly His Ile Pro Leu Thr Asp Val Pro Ser Leu Phe Pro Arg Phe
               180                 185                 190
   Leu Pro Val Pro Glu Glu Ser Gly Ala His Val Val Leu Leu Gly Gly
           195                 200                 205
   Ser Arg Leu Leu Met Ala Ala Ser Ala Pro Lys Thr Ala Glu Leu Leu
       210                 215                 220
   Ala Asp Leu Gly His Glu Pro Val Leu Val Asp Ile Gly Glu Phe Glu
   225                 230                 235                 240
   Lys Leu Glu Gly Cys Val Thr Cys Leu Ser Val Arg Leu Arg Glu Leu
                   245                 250                 255
   Tyr Asp
(2) INFORMATION FOR SEQ ID NO: 7:
   (i) SEQUENCE CHARACTERISTICS:
      (A) LENGTH: 765 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 7:
   ATGTTCAAGC ACATCATCGC TCGCACGCCC GCCCGCAGCC TGGTCGACGG CCTGACCTCC      60
   AGCCACCTCG GCAAGCCGGA CTACGCCAAG GCCCTGGAGC AGCACAACGC CTACATCCGC 120
   GCCTTGCAGA CCTGCGACGT GGACATCACC CTGCTGCCGC CGGACGAACG CTTCCCCGAC 180
   TCGGTGTTCG TCGAGGACCC GGTGCTCTGC ACCTCGCGCT GCGCCATCAT CACCCGCCCC 240
   GGCGCCGAAT CGCGGCGCGG CGAGACCGAG ATCATCGAGG AAACCGTGCA GCGCTTCTAT 300
   CCGGGCAAGG TCGAGCGCAT CGAGGCACCC GGCACGGTGG AAGCCGGCGA CATCATGATG 360
   GTCGGCGACC ACTTCTACAT CGGCGAATCG GCCCGCACCA ACGCCGAGGG CGCCCGGCAG 420
   ATGATCGCGA TCCTGGAGAA ACATGGCCTC AGCGGCTCGG TGGTGCGCCT GGAAAAGGTC 480
   CTGCACCTGA AGACCGGGCT CGCCTACCTG GAACACAACA ACCTGCTGGC CGCCGGCGAG 540
   TTCGTCAGCA AGCCGGAGTT CCAGGACTTC AACATCATCG AGATCCCCGA AGAGGAGTCC 600
   TACGCCGCCA ACTGCATCTG GGTCAACGAA AGGGTGATCA TGCCCGCCGG CTATCCCCGG 660
   ACCCGCGAGA AGATCGCCCG CCTCGGCTAC CGGGTGATCG AGGTGGACAC CTCCGAATAT 720
   CGCAAGATCG ACGGCGGCGT CAGTTGCATG TCGCTGCGCT TCTGA                765
(2) INFORMATION FOR SEQ ID NO: 8:
   (i) SEQUENCE CHARACTERISTICS:
      (A) LENGTH: 254 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 8:
   Met Phe Lys His Ile Ile Ala Arg Thr Pro Ala Arg Ser Leu Val Asp
   1               5                   10                  15
   Gly Leu Thr Ser Ser His Leu Gly Lys Pro Asp Tyr Ala Lys Ala Leu
               20                  25                  30
   Glu Gln His Asn Ala Tyr Ile Arg Ala Leu Gln Thr Cys Asp Val Asp
           35                  40                  45
   Ile Thr Leu Leu Pro Pro Asp Glu Arg Phe Pro Asp Ser Val Phe Val
       50                  55                  60
   Glu Asp Pro Val Leu Cys Thr Ser Arg Cys Ala Ile Ile Thr Arg Pro
   65                  70                  75                  80
   Gly Ala Glu Ser Arg Arg Gly Glu Thr Glu Ile Ile Glu Glu Thr Val
                   85                  90                  95
   Gln Arg Phe Tyr Pro Gly Lys Val Glu Arg Ile Glu Ala Pro Gly Thr
               100                 105                 110
   Val Glu Ala Gly Asp Ile Met Met Val Gly Asp His Phe Tyr Ile Gly
           115                 120                 125
   Glu Ser Ala Arg Thr Asn Ala Glu Gly Ala Arg Gln Met Ile Ala Ile
       130                 135                 140
   Leu Glu Lys His Gly Leu Ser Gly Ser Val Val Arg Leu Glu Lys Val
   145                 150                 155                 160
   Leu His Leu Lys Thr Gly Leu Ala Tyr Leu Glu His Asn Asn Leu Leu
                   165                 170                 175
   Ala Ala Gly Glu Phe Val Ser Lys Pro Glu Phe Gln Asp Phe Asn Ile
               180                 185                 190
   Ile Glu Ile Pro Glu Glu Glu Ser Tyr Ala Ala Asn Cys Ile Trp Val
           195                 200                 205
   Asn Glu Arg Val Ile Met Pro Ala Gly Tyr Pro Arg Thr Arg Glu Lys
       210                 215                 220
   Ile Ala Arg Leu Gly Tyr Arg Val Ile Glu Val Asp Thr Ser Glu Tyr
   225                 230                 235                 240
   Arg Lys Ile Asp Gly Gly Val Ser Cys Met Ser Leu Arg Phe
                   245                 250
(2) INFORMATION FOR SEQ ID NO: 9:
   (i) SEQUENCE CHARACTERISTICS:
      (A) LENGTH: 1257 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 9:
   ATGAGCACGG AAAAAACCAA ACTTGGCGTC CACTCCGAAG CCGGCAAACT GCGCAAAGTG     60
   ATGGTCTGCT CGCCCGGACT CGCCCACCAG CGCCTGACCC CGAGCAACTG CGACGAGTTG    120
   CTGTTCGACG ACGTGATCTG GGTGAACCAG GCCAAGCGCG ACCACTTCGA CTTCGTCACC    180
   AAGATGCGCG AGCGCGGCAT CGACGTCCTC GAGATGCACA ATCTGCTGAC CGAGACCATC 240
   CAGAACCCGG AAGCGCTGAA GTGGATCCTC GATCGCAAGA TCACCGCCGA CAGCGTCGGC 300
   CTGGGCCTGA CCAGCGAGCT GCGCTCCTGG CTGGAGAGCC TGGAGCCGCG CAAGCTGGCC 360
   GAGTACCTGA TCGGCGGCGT CGCCGCTGAC GACCTGCCCG CCAGCGAAGG CGCCAACATC 420
   CTCAAGATGT ACCGCGAGTA CCTGGGCCAT TCCAGCTTCC TGCTGCCGCC GTTGCCGAAC 480
   ACCCAGTTCA CCCGCGACAC CACTTGCTGG ATCTACGGCG GCGTGACCCT GAACCCGATG 540
   TACTGGCCGG CGCGACGACA GGAAACCCTG CTGACCACCG CCATCTACAA GTTCCACCCC 600
   GAGTTCGCCA ACGCCGAGTT CGAGATCTGG TACGGCGACC CGGACAAGGA CCACGGCTCC 660
   TCGACCCTGG AAGGCGGCGA CGTGATGCCG ATCGGCAACG GCGTGGTCCT GATCGGCATG 720
   GGCGAGCGCT CCTCGCGCCA GGCCATCGGT CAGGTCGCCC AGTCGCTGTT CGCCAAGGGC 780
   GCCGCCGAGC GGGTGATCGT CGCCGGCCTG CCGAAGTCCC GCGCCGCGAT GCACCTGGAC 840
   ACCGTGTTCA GCTTCTGCGA CCGCGACCTG GTCACGGTCT TCCCGGAAGT GGTCAAGGAA 900
   ATCGTGCCCT TCAGCCTGCG CCCCGATCCG AGCAGCCCCT ACGGCATGAA CATCCGCCGC 960
   GAGGAGAAAA CCTTCCTCGA AGTGGTCGCC GAATCCCTCG GCCTGAAGAA ACTGCGCGTG 1020
   GTCGAGACCG GCGGCAACAG CTTCGCCGCC GAGCGCGAGC AATGGGACGA CGGTAACAAC 1080
   GTGGTCTGCC TGGAGCCGGG CGTGGTGGTC GGCTACGACC GCAACACCTA CACCAACACC 1140
   CTGCTGCGCA AGGCCGGCGT CGAGGTCATC ACCATCAGCG CCAGCGAACT GGGTCGCGGT 1200
   CGCGGCGGCG GCCACTGCAT GACCTGCCCG ATCGTCCGCG ACCCGATCGA CTACTGA 1257
(2) INFORMATION FOR SEQ ID NO: 10:
   (i) SEQUENCE CHARACTERISTICS:
      (A) LENGTH: 418 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 10:
   Met Ser Thr Glu Lys Thr Lys Leu Gly Val His Ser Glu Ala Gly Lys
   1 5 10 15
   Leu Arg Lys Val Met Val Cys Ser Pro Gly Leu Ala His Gln Arg Leu
               20                  25                  30
   Thr Pro Ser Asn Cys Asp Glu Leu Leu Phe Asp Asp Val Ile Trp Val
           35                  40                  45
   Asn Gln Ala Lys Arg Asp His Phe Asp Phe Val Thr Lys Met Arg Glu
       50                  55                  60
   Arg Gly Ile Asp Val Leu Glu Met His Asn Leu Leu Thr Glu Thr Ile
   65                  70                  75                  80
   Gln Asn Pro Glu Ala Leu Lys Trp Ile Leu Asp Arg Lys Ile Thr Ala
                   85                  90                  95
   Asp Ser Val Gly Leu Gly Leu Thr Ser Glu Leu Arg Ser Trp Leu Glu
               100                 105                 110
   Ser Leu Glu Pro Arg Lys Leu Ala Glu Tyr Leu Ile Gly Gly Val Ala
           115                 120                 125
   Ala Asp Asp Leu Pro Ala Ser Glu Gly Ala Asn Ile Leu Lys Met Tyr
       130                 135                 140
   Arg Glu Tyr Leu Gly His Ser Ser Phe Leu Leu Pro Pro Leu Pro Asn
   145                 150                 155                 160
   Thr Gln Phe Thr Arg Asp Thr Thr Cys Trp Ile Tyr Gly Gly Val Thr
                   165                 170                 175
   Leu Asn Pro Met Tyr Trp Pro Ala Arg Arg Gln Glu Thr Leu Leu Thr
               180                 185                 190
   Thr Ala Ile Tyr Lys Phe His Pro Glu Phe Ala Asn Ala Glu Phe Glu
           195                 200                 205
   Ile Trp Tyr Gly Asp Pro Asp Lys Asp His Gly Ser Ser Thr Leu Glu
       210                 215                 220
   Gly Gly Asp Val Met Pro Ile Gly Asn Gly Val Val Leu Ile Gly Met
   225                 230                 235                 240
   Gly Glu Arg Ser Ser Arg Gln Ala Ile Gly Gln Val Ala Gln Ser Leu
                   245                 250                 255
   Phe Ala Lys Gly Ala Ala Glu Arg Val Ile Val Ala Gly Leu Pro Lys
               260                 265                 270
   Ser Arg Ala Ala Met His Leu Asp Thr Val Phe Ser Phe Cys Asp Arg
           275                 280                 285
   Asp Leu Val Thr Val Phe Pro Glu Val Val Lys Glu Ile Val Pro Phe
       290                 295                 300
   Ser Leu Arg Pro Asp Pro Ser Ser Pro Tyr Gly Met Asn Ile Arg Arg
   305                 310                 315                 320
   Glu Glu Lys Thr Phe Leu Glu Val Val Ala Glu Ser Leu Gly Leu Lys
                   325                 330                 335
   Lys Leu Arg Val Val Glu Thr Gly Gly Asn Ser Phe Ala Ala Glu Arg
               340                 345                 350
   Glu Gln Trp Asp Asp Gly Asn Asn Val Val Cys Leu Glu Pro Gly Val
           355                 360                 365
   Val Val Gly Tyr Asp Arg Asn Thr Tyr Thr Asn Thr Leu Leu Arg Lys
       370                 375                 380
   Ala Gly Val Glu Val Ile Thr Ile Ser Ala Ser Glu Leu Gly Arg Gly
   385                 390                 395                 400
   Arg Gly Gly Gly His Cys Met Thr Cys Pro Ile Val Arg Asp Pro Ile
                   405                 410                 415
   Asp Tyr
(2) INFORMATION FOR SEQ ID NO: 11:
   (i) SEQUENCE CHARACTERISTICS:
      (A) LENGTH: 1014 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 11:
   ATGTATCAAT GGAAAATACG CAACGACCAT CGTTTGATTG TGAAATCAGA GCCAAATATC      60
   GTTGGTTTAT GACGGATTCC TACGTCGCTG CTGCCCGTCT AGGGTCACCT GCACGCCGCA     120
   CCCCCCGGAC GCGGCGGTAT GCAATGACCC CGCCGGCCTT CTTTGCCGTC GCATACGCGA     180
   TCAACCCCTG GATGGACGTC ACCGCGCCAG TCGACGTCCA AGTCGCGCAA GCACAGTGGG     240
   AGCACCTCCA CCAGACCTAT CTTCGGCTAG GCCACAGCGT GGATCTGATC GAGCCCATTT     300
   CCGGGTTACC GGACATGGTG TACACCGCCA ACGGTGGGTT CATCGCGCAC GACATCGCCG     360
   TGGTCGCCCG GTTCCGGTTC CCCGAACGAG CTGGTGAGTC TAGAGCCTAT GCCAGCTGGA 420
   TGTCCTCGGT CGGATATCGC CCGGTGACCA CCCGCCACGT CAACGAGGGA CAGGGCGACC 480
   TGCTGATGGT TGGCGAAAGG GTGTTGGCGG GCTACGGCTT TCGCACAGAC CAGCGCGCAC 540
   ACGCCGAAAT CGCCGCGGTG CTTGGTCTGC CGGTGGTCTC CCTCGAGTTG GTCGACCCAC 600
   GGTTCTATCA CCTGGACACC GCGCTGGCCG TGCTCGACGA CCACACGATC GCCTACTACC 660
   CGCCGGCGTT CAGTACGGCA GCGCAGGAAC AGTTGTCGGC GCTGTTCCCC GACGCGATTG 720
   TGGTCGGCAG TGCCGACGCG TTCGTGTTCG GACTCAACGC CGTCTCTGAC GGTCTGAACG 780
   TAGTGCTTCC GGTCGCGGCC ATGGGTTTTG CGGCGCAGTT ACGCGCAGCC GGCTTCGAGC 840
   CGGTCGGTGT CGATCTGTCC GAGCTGCTCA AGGGCGGCGG TTCCGTCAAG TGCTGCACGC 900
   TGGAGATACA CCCATGACAA ATCTCGCGGA TGCCACTCAG GCCACTATGG CACTGGTCGA 960
   AAGGCATGCA GCGCACAATT ATTCGCCGCT GCCTGTGGTG GCGGCCAGCG CTGA 1014
(2) INFORMATION FOR SEQ ID NO: 12:
   (i) SEQUENCE CHARACTERISTICS:
      (A) LENGTH: 305 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 12:
   Asn Val Ser Met Glu Asn Thr Gln Arg Pro Ser Phe Asp Cys Glu Ile
   1               5                   10                  15
   Arg Ala Lys Tyr Arg Trp Phe Met Thr Asp Ser Tyr Val Ala Ala Ala
               20                  25                  30
   Arg Leu Gly Ser Pro Ala Arg Arg Thr Pro Arg Thr Arg Arg Tyr Ala
           35                  40                  45
   Met Thr Pro Pro Ala Phe Phe Ala Val Ala Tyr Ala Ile Asn Pro Trp
       50                  55                  60
   Met Asp Val Thr Ala Pro Val Asp Val Gln Val Ala Gln Ala Gln Trp
   65                  70                  75                  80
   Glu His Leu His Gln Thr Tyr Leu Arg Leu Gly His Ser Val Asp Leu
                   85                  90                  95
   Ile Glu Pro Ile Ser Gly Leu Pro Asp Met Val Tyr Thr Ala Asn Gly
               100                 105                 110
   Gly Phe Ile Ala His Asp Ile Ala Val Val Ala Arg Phe Arg Phe Pro
           115                 120                 125
   Glu Arg Ala Gly Glu Ser Arg Ala Tyr Ala Ser Trp Met Ser Ser Val
       130                 135                 140
   Gly Tyr Arg Pro Val Thr Thr Arg His Val Asn Glu Gly Gln Gly Asp
   145                 150                 155                 160
   Leu Leu Met Val Gly Glu Arg Val Leu Ala Gly Tyr Gly Phe Arg Thr
                   165                 170                 175
   Asp Gln Arg Ala His Ala Glu Ile Ala Ala Val Leu Gly Leu Pro Val
               180                 185                 190
   Val Ser Leu Glu Leu Val Asp Pro Arg Phe Tyr His Leu Asp Thr Ala
           195                 200                 205
   Leu Ala Val Leu Asp Asp His Thr Ile Ala Tyr Tyr Pro Pro Ala Phe
       210                 215                 220
   Ser Thr Ala Ala Gln Glu Gln Leu Ser Ala Leu Phe Pro Asp Ala Ile
   225                 230                 235                 240
   Val Val Gly Ser Ala Asp Ala Phe Val Phe Gly Leu Asn Ala Val Ser
                   245                 250                 255
   Asp Gly Leu Asn Val Val Leu Pro Val Ala Ala Met Gly Phe Ala Ala
               260                 265                 270
   Gln Leu Arg Ala Ala Gly Phe Glu Pro Val Gly Val Asp Leu Ser Glu
           275                 280                 285
   Leu Leu Lys Gly Gly Gly Ser Val Lys Cys Cys Thr Leu Glu Ile His
       290                 295                 300
   Pro
   305